# EUROPEAN PATENT SPECIFICATION

(11) **EP 3 935 390 B1**
(45) Date of publication and mention of the grant of the patent: **01.05.2024**
(21) Application number: 20707156.4
(22) Date of filing: 05.03.2020
(51) Int. Cl.: G01N 33/543

(54) **SENSOR FOR SINGLE PARTICLE DETECTION**
SENSOR ZUR ERKENNUNG EINZELNER PARTIKEL
CAPTEUR POUR LA DÉTECTION DE PARTICULES INDIVIDUELLES

(30) Priority: 05.03.2019 EP 19160822; 16.04.2019 EP 19169429
(43) Date of publication of application: 12.01.2022
(73) Proprietor: ECsens B. V., 7522 NH Enschede (NL)
(72) Inventor: MATHEW, Dilu George, 7500 AE Enschede (NL); BEEKMAN, Pepijn, 7500 AE Enschede (NL); VAN DER WIEL, Wilfred Gerard, 7500 AE Enschede (NL); LEMAY, Serge Joseph Guy, 7500 AE Enschede (NL)
(74) Representative: EDP Patent Attorneys B.V.
(86) International application number: PCT/EP2020/055945
(87) International publication number: WO 2020/178408

(56) References cited:
- SUN ALEXANDER C ET AL: "High-Density Redox Amplified Coulostatic Discharge-Based Biosensor Array", IEEE JOURNAL OF SOLID-STATE CIRCUITS, IEEE SERVICE CENTER, PISCATAWAY, NJ, USA, vol. 53, no. 7, 1 July 2018 (2018-07-01), pages 2054-2064, XP011686426, ISSN: 0018-9200, DOI: 10.1109/JSSC.2018.2820705 [retrieved on 2018-07-02]
- SUN ALEXANDER ET AL: "A 64x64 high-density redox amplified coulostatic discharge-based biosensor array in 180nm CMOS", ESSCIRC 2017 - 43RD IEEE EUROPEAN SOLID STATE CIRCUITS CONFERENCE, IEEE, 11 September 2017 (2017-09-11), pages 368-371, XP033246253, DOI: 10.1109/ESSCIRC.2017.8094602
- TUGBA KILIC ET AL: "Label-free detection of hypoxia-induced extracellular vesicle secretion from MCF-7 cells", SCIENTIFIC REPORTS, vol. 8, no. 1, 20 June 2018 (2018-06-20), XP055623780, DOI: 10.1038/s41598-018-27203-9
- ZHOU QING ET AL: "Development of an aptasensor for electrochemical detection of exosomes", METHODS, vol. 97, 15 March 2016 (2016-03-15), pages 88-93, XP029460665, ISSN: 1046-2023, DOI: 10.1016/J.YMETH.2015.10.012
- KSENIIA BORIACHEK ET AL: "An amplification-free electrochemical detection of exosomal miRNA-21 in serum samples", ANALYST, vol. 143, no. 7, 1 January 2018 (2018-01-01), pages 1662-1669, XP055623745, UK ISSN: 0003-2654, DOI: 10.1039/C7AN01843F
- RONGRONG HUANG ET AL: "A Sensitive Aptasensor Based on a Hemin/G-Quadruplex-Assisted Signal Amplification Strategy for Electrochemical Detection of Gastric Cancer Exosomes", SMALL, vol. 15, no. 19, 8 April 2019 (2019-04-08) , page 1900735, XP055623103, DE ISSN: 1613-6810, DOI: 10.1002/smll.201900735

## Description

### FIELD OF THE INVENTION

The invention relates to a sensor for sensing a predetermined particle, a device for analyzing a fluid, and a method for analyzing a fluid.

### BACKGROUND OF THE INVENTION

Processes for detection of tumor cells in bodily fluids are known in the art. WO2017034836, e.g., describes methods of determining whether a circulating tumor cell (CTC) marker, e.g., a CTC or fragment thereof, is present in a sample, such as a blood sample, are provided. Aspects of the methods include flow cytometrically assaying a fluorescently labeled sample that has been fluorescently labeled with a fluorescently labeled chlorotoxin binding member to determine whether a CTC marker is present in the sample.

Sun et al., "High-Density Redox Amplified Coulostatic Discharge-Based Biosensor Array", in IEEE JOURNAL OF SOLID-STATE CIRCUITS, 2018, vol. 53(7), pp 2054-2064, describes a high-density 4,096-pixel electrochemical biosensor array in 180-nm CMOS It uses a coulostatic discharge sensing technique and interdigitated electrode (IDE) geometry to reduce both the complexity and size of the readout circuitry. Each biopixel contains an interdigitated microelectrode with a 13-aA low-leakage readout circuit directly underneath. The detection of anti-Rubella and anti-Mumps antibodies in human serum is demonstrated.

Sun et al., "A 64x64 high-density redox amplified coulostatic discharge-based biosensor array in 180nm CMOS", ESSCIRC 2017 - 43RD IEEE EUROPEAN SOLID STATE CIRCUITS CONFERENCE, pp 368-371, further describes the design of density 4,096-pixel electrochemical biosensor array in 180nm CMOS for biomedical applications that require multiple analyte detection from small (5µL) samples. Each pixel of the array contains an exposed 45×45µm² interdigitated micro-electrode surrounded by a ~9pL nanowell.

Kilic et al., "Label-free detection of hypoxia-induced extracellular vesicle secretion from MCF-7 cells", SCIENTIFIC REPORTS, 2018, vol. 8(1), describes a label free electrochemical sensor to measure extracellular vesicle secretion. The sensor design includes two consecutive steps; i) Au electrode surface functionalization for anti-CD81 Antibody and ii) EVs capture. The label-free detection of EVs was done via Differential Pulse Voltammetry (DPV) and Electrochemical Impedance Spectroscopy (EIS). The working linear range for the sensor was 10² -10⁹ EVs/ml with an LOD 77 EVs/mL and 379 EVs/ml for EIS and DPV based detection.

Zhou et al., "Development of an aptasensor for electrochemical detection of exosomes", METHODS, 2016, vol. 97, pp 88-93, describes an aptamer-based electrochemical biosensor for quantitative detection of exosomes. Aptamers specific to exosome transmembrane protein CD63 were immobilized onto gold electrode surfaces and incorporated into a microfluidic system. Probing strands pre-labeled with redox moieties were hybridized onto aptamer molecules anchored on the electrode sur-face. In the presence of exosomes these beacons released probing strands with redox reporters causing electrochemical signal to decrease.

Boriacheck et al., "An amplification-free electrochemical detection of exosomal miRNA-21 in serum samples", ANALYST,2018, vol. 143(7), pp 1662-1669, describes an electrochemical approach for the detection of cancer-derived exosomal miRNAs in human serum samples by selectively isolating the target miRNA using magnetic beads pre-functionalized with capture probes and then directly adsorbing the targets onto a gold electrode surface. The level of adsorbed miRNA is detected electrochemically in the presence of an (Fe(CN)₆]^{4-/3-} redox system.

Rongrong et al., "A Sensitive Aptasensor Based on a Hemin/G-Quadruplex-Assisted Signal Amplification Strategy for Electrochemical Detection of Gastric Cancer Exosomes", SMALL, 2019, vol. 15 page 1900735 describes a label free aptasensor for specific detection of gastric cancer exosomes. The platform contains an anti-CD63 antibody modified gold electrode and a gastric cancer exosome specific aptamer. The aptamer is linked to a primer sequence that is complementary to a G-quadruplex circular template. The presence of target exosomes could trigger rolling circle amplification and produce multiple G-quadruplex units.

### SUMMARY OF THE INVENTION

A challenge in cancer patient care is to monitor the response to treatment. Nowadays, most of the established approaches use tissue staining, which implies their invasive collection through, e.g., biopsies of the primary tumor. Patient follow-up therefore requires serial biopsies, making it very unpleasant for the patient. Alternatively, in vivo imaging approaches using MRI and PET-CT are used, with an essential limitation that they can only detect tumors of at least 1 cm. Moreover, high cost, radiation problems (PET-CT), and/or allergic reactions render these tests unsuitable for repeated use. Nonetheless, more frequent monitoring of the patient to study the treatment efficacy is crucial for making a more accurate prognosis and prescribing personalized treatment.

Currently, circulating tumor cells (CTCs) are being used for liquid biopsies. These CTCs, though, are much less available in blood than e.g. tumor-derived extracellular vesicles (tdEVs). The ratio of tdEVs to CTCs in blood is about 10³. Hence, analysis based on the detection of tdEV may give warnings or status information at an earlier stage than analysis based on CTC detection. The relatively high abundance of tdEVs compared to CTCs makes tdEVs more appealing for cancer disease management. More tdEVs are present in a droplet of blood (∼50 µl) than there are CTCs in 7.5 ml of blood (the standard sample volume used in CellSearch^{®} - to date the only FDA-approved technology for CTC analysis for cancer diagnosis, prognosis, and patient management for certain types of cancer). It may be concluded that much less sample is required to reliably confirm presence or absence of cancer markers based on tdEV detection. To enable accurate quantification of tdEVs from patient blood, an ultra-sensitive and ultra-selective device is required, to detect individual submicron particles at low concentration (with ~10³ EVs ml⁻¹ resolution).

EVs are small (50 nm - 1 µm), membrane-enclosed carriers, which are produced by all cells. Nonetheless, the limit of detection (LOD) of tdEVs using state-of-the-art techniques is several orders of magnitude higher than their clinically relevant concentrations (which is about 10⁴ tdEVs/ml) in blood. The disadvantages found in cancer diagnostics may also be found in diagnostics/analysis related to other medical, physical, biological and/or (bio)chemical fields.

Hence, it is an aspect of the invention to provide an alternative sensor for sensing a (predetermined) particle in a fluid, which further at least partly obviates one or more of above-described drawbacks. It is a further aspect to provide a device (especially comprising a sensor according to the invention) for analyzing a fluid (comprising a (predetermined) particle), which further at least partly obviates one or more of above-described drawbacks. In a further aspect, the invention provides a system for analyzing a fluid, comprising the device described herein, which further at least partly obviates one or more drawbacks of prior art systems for analyzing a fluid. The invention further provides a method for analyzing a fluid, which further at least partly obviates one or more of above-described drawbacks. In the method, the system of the invention is used. The present invention may have as object to overcome or ameliorate at least one of the disadvantages of the prior art, or to provide a useful alternative.

The sensor, device, system and method of the invention may enable detection of ultra-low concentrations of (biological) particles. The (predetermined) particle comprises a biological particle, e.g. a tdEV. The fluid may e.g. comprise blood. Particles, such as (tumor-derived) extracellular vesicles, e.g., may be detected on a single particle level. The sensor (and method) may detect single particles without calibration. Furthermore, the method may enable fast measurements thanks to an electrophoretic attraction of the particles to the electrode surface. Based on electrophoretic attraction, particles in the fluid may be attracted to the electrode (and sensed) a factor of about several orders of magnitude faster compared to diffusive transport of the particle alone. The sensor, device, system, and method further may allow distinction of specific and non-specific interactions of particles on/at the surface, thereby reducing the amount of false positive detections. Moreover, the method is a label-free method supporting real-time analysis. The method may provide direct and real-time information about the presence (especially including the number) of specific particles (especially in a complex medium). The method may determine discrete events (presence/absence of the (one or more) particle(s)) and may provide quantitative information directly from the measured current over time signal. The discrete events especially refer to discrete interaction events of an individual particle with the electrode. The invention may e.g. be used for both highly sensitive and especially selective cancer marker detection in blood. In (other) embodiments, the invention may e.g. be used for both highly sensitive and especially selective cancer marker detection in blood (plasma), urine, semen (seminal fluid), vaginal secretions, cerebrospinal fluid (CSF), synovial fluid, pleural fluid (pleural lavage), pericardial fluid, peritoneal fluid, amniotic fluid, saliva, nasal fluid, otic fluid, gastric fluid, breast milk, etc.

Furthermore, the device and system may be cheaper than prior art solutions, and may be configured as a portable device/system. The system may be a handheld system and easy to use enabling fast and simple diagnostics at laboratories and clinics. The invention especially provides a robust and easy-to-use, non-invasive biosensing platform. The invention may be used in relation to all kinds of biological particles in a fluid. The invention is used, in relation to biological particles in a fluid such as in bodily fluids or e.g. in another kind of fluid present in nature. The concept of the invention is especially explained based on a biological particle. However, the concept may also be applied in relation to non-biological (inorganic) particles. The fluid not necessarily is a bodily fluid. Examples of the particles are e.g. extracellular vesicles, viruses, bacteria, (poly)peptides, proteins, hormones, biopolymers (like DNA, RNA), enzymes, chemicals, drugs, particles functionalized (modified) with DNA, and/or RNA. Herein the term "a particle modified with DNA (or RNA)" especially relates to a particle having a DNA (or RNA) linked to the particle. As such the particle may be functionalized with the DNA (or RNA). A particle that is functionalized with DNA or RNA may function as a substrate for the RNA or DNA (RNA or DNA may be the actual analyte to detect).

In a first aspect, the invention provides a sensor for sensing a predetermined particle in a fluid as further defined in the accompanying claims. The fluid especially comprises a liquid (fluid). The fluid further comprises a redox mediator. The fluid may further comprise an electrolyte. The sensor comprises an electrode and a recognition element (for the (predetermined) particle / able to recognize the (predetermined) particle). The electrode comprises an electrode face. The electrode face is configured accessible to the fluid and also (accessible) to the (predetermined) particle in the fluid. The electrode face is further configured accessible to a redox mediator in the fluid. The recognition element is configured (and/or selected) for (at least temporarily) selectively binding (or engaging) with the predetermined particle. Such binding may be reversible. As a result of the binding, especially as a result of the presence of the particle, access of the redox mediator to the electrode face is limited (during binding of the particle with the recognition element). The recognition element is configured to limit access of the redox mediator to the electrode face during the binding of the predetermined particle with the recognition element (see further below).

The term "recognition element" especially relates to an element that binds to (engages with) (a part of) a determined particle. The recognition element may hold the determined particle and/or may attach to the particle. The recognition element may have a characteristic that is complementary to (or "matches") a characteristic of a (part or location) of the particle and may therefore "recognize" (at least a part of) the particle and engage with (the at least part of) the particle. As such, the recognition element may also be called an "engagement element". The recognition element comprises a biological recognition element (for the predetermined particle). Furthermore, also the terms "recognition biomolecule" and "bioreceptor" may be used to address a biological recognition element. The recognition element comprises a biological recognition element that may bind to a biological particle. An example of such biological recognition element is an antibody (see further below). The recognition element may bind to a specific location of the particle. The particle may therefore especially comprise at least one (specific) location being able to bind (or engage) with the recognition element.

Herein, the term" binding" especially relates to at least temporarily having an interaction between the particle and the electrode and at least temporarily holding the particle to the recognition element. Binding especially relates to physical binding and/or chemical binding. The predetermined particle and the recognition element may form a complex during binding (such as an antibody-antigen complex). In specific embodiments multiple particles may be bound to (multiple recognition elements at) the electrode, together at least partly blocking the electrode face. The particle(s) (when bound to the recognition element) at least temporarily partly block a (diffusive) transport of the redox mediator to the electrode face. In specific embodiments, only a single predetermined particle may be bound to the electrode (and especially block the electrode for further particles during binding). In further embodiments, multiple particles may be bound to the electrode. In embodiments, the electrode may comprise equal to or less than 1000 engagement elements, such as equal to or less than 500 engagement elements, especially equal to or less than 100 engagement element, such as equal to or less than 20 engagement elements (such as 1 (or two or three) engagement element). In embodiments, the electrode may comprise more than 1000 engagement elements, such as up to 10000 or even up to 50000. In specific embodiments, the electrode (face) comprises 100-10000 engagement elements.

Especially, a size or characteristic dimension (see below) of the electrode (face) (and a number and/or distribution of engagement elements (over the electrode)) may be selected to bind up to about 10,000 particles, such as up to about 2,000, like about at maximum 1000, such as not more than about 500 particles (simultaneously). More especially, a size or characteristic dimension (see below) of the electrode (face) (and a number and/or distribution of engagement elements (over the electrode)) may be selected to bind a maximum of 200, especially of 100, such as of 50, especially a maximum of 20 particles (simultaneously). In embodiments the electrode may bind only 1 particle (based on the size of the electrode and the particles and/or e.g. the presence of only 1 engagement element). If multiple particles are (simultaneously) bound to the electrode, the binding and releasing of a single particle may (still) be observed based on a changing limitation of the transport of the redox mediator to the electrode. Such change may show discrete changes in the measured current vs time (signal) and especially allows detection of single (individual) particles. For instance, experimentally is has been shown that in embodiment comprising a circular electrode with a diameter of 200 nm single particles of 8 nm may be detected. Hence, the method especially comprises single (individual) particle detection. Moreover, the sensor is especially configured for single (individual) particle detection.

Herein, a period during which the particle is bound to the recognition element may also be referred to as "an engagement period". The term "engagement period" especially relates to a determined time period or duration. The engagement period is a result of many parameters and may e.g. be affected by the affinity between the particle and the recognition element. Moreover, the engagement period for a particle bound to a plurality of recognition elements (all at the same electrode face) may be substantially longer than the one for a further particle bound (only) to one recognition element. If the fluid is flowing in (through) the sensor, the flowing fluid may induce a force at the particle, liberating the particle from the recognition element again (therewith reducing the engagement period). The engagement period may further depend on electrode characteristics, such as a shape and/or size of the electrode face, a coating at the electrode, the (number of) recognition element, etc.. Yet, even for the same particle and recognition element combination, the engagement period may vary over a couple of orders of magnitude.

The engagement period, though, is normally especially longer than a time required for a particle to move (based on a diffusive transport) over (and to shield) the electrode face. In embodiments, it may be clear from the measured current versus time data that a particle selectively binds with the recognition element. Especially, based on multiple encounters of one or more particles and an electrode (face) a differentiation between a particle that is at least temporarily (selectively) bound to a recognition element and a particle that is not selectively bound to the recognition element (but may e.g. be located at or before the electrode face) may be clearer. Statistical analysis may further facilitate, in differentiating between the predetermined particle and a (non-specific) particle (see further below).

The engagement period for specific interactions may be in the range of hundreds of milliseconds. Yet, the engagement period may also be smaller or larger. In embodiments, the determined particle may be bound to the recognition element until it is flushed away from the element, e.g. by a separation fluid (see below). The engagement period may at least be 10 microseconds such as at least 100 microseconds. The engagement period may especially comprise at least 10 milliseconds, such as at least 25 milliseconds, especially at least 50 milliseconds. The engagement period (for a specific particle) may especially be equal to or less than 10,000 milliseconds, such as equal to or less than 5000 milliseconds, especially equal to or less than 2500 milliseconds. The engagement period may in further embodiments be at least 10 sec., such as at least 25 sec, especially at least 1 min. such as up to (substantially) indefinite when the binding is irreversible. A typical "half-life" of a specific combination (complex) of the (predetermined) particle with the engagement element may be about 0.5 second to several seconds. Yet, a variation around the half-life may be about two orders of magnitude depending on the affinity of the particle for the recognition element.

A non-predetermined (or "random") particle may perhaps only (partly) limit access of the redox mediator to the electrode face during a few milliseconds or less. However, the non-predetermined particle may also limit access for a longer period of time, depending e.g. on the type of particle, the electrode characteristics, a motion of the fluid, etc., a coating (see further below) may be configured for reducing that period of time.

The engagement period is especially a result of the association and dissociation constants of the binding "reactions" (complex forming) between the predetermined particle and the recognition element. These constants may again be a function of the ionic strength of the fluid. The association constant may also be referred to as the affinity constant. Especially, the recognition element is selected such that the ratio of the respective association constant to the dissociation constant is larger than 1, especially larger than 10. The constant may typically be lower than 10000.

The recognition element is essentially functionally coupled to the electrode. The recognition element is not necessarily physically connected to the electrode; the recognition element and the electrode function together and especially define a functional unit. The recognition element may e.g. be arranged over the electrode face, or at the electrode face. The recognition element may, e.g., be comprised by or linked to a coating at the electrode face (see below). Therefore, herein this functional unit and the respective parts may also be referred to with phrases like "the electrode face and (or including) the recognition element", "the recognition element of the electrode (face)", etc. Furthermore, it may be described that "the electrode (face) comprises the recognition element". The last phrase may indicate that the recognition element is (directly or indirectly) attached to the electrode (face). It may also relate to a situation in which the recognition element is not in direct physical contact with the electrode face. Moreover, it especially indicates that when a predetermined particle is bound to the recognition element, the transport of the redox mediator to the respective electrode (face) is at least limited (by the combination/complex of the respective recognition element and the predetermined particle). If the sensor, device or system comprises more than one electrode, each electrode (face) may form a functional unit with a respective recognition element. It may be described that each electrode (face) comprises (is functionally coupled to) a respective recognition element (of the sensor).

The term "recognition element" may further relate to a plurality of (different) recognition elements. In embodiments, a plurality of (especially the same) recognition elements are configured at the same electrode face. A plurality of recognition elements may e.g. bind with the same (predetermined) particle (if the particle comprises more than one location being able to bind with the recognition element) and/or with more than one (predetermined) particle. Hence, a single electrode (face) may form a functional unit with more than one recognition element. In embodiments, e.g., each electrode comprises a plurality of recognition elements, such as described above. Each recognition element is functionally coupled to only one electrode (face).

To sense a specific or predetermined particle, the recognition element may be configured to selectively bind (interact) with the predetermined particle. In embodiments, the sensor may be configured for sensing a (specific) biological particle, and especially the recognition element may comprise a biological recognition element, especially an antibody, for the biological particle (see further below).

The expressions "a (biological) recognition element for the (biological) particle" and "an antibody for the biological particle" especially relate to a combination of the (biological) recognition element, such as the antibody, and the (biological) particle, wherein the (biological) particle has a high affinity for (biological) recognition element. The biological particle may for instance comprise a particular binding site or epitope, that may bind to a (corresponding) particular binding site or paratope of the recognition element (antibody). The paratope is especially specific for the epitope. The affinity is especially a chemical affinity and relating to an affinity between a biological particle and a (biological) recognition element. The (predetermined) particle may comprise a plurality of binding sites for the recognition element. Herein, also the terms "antigen" or "biomarker" may be used to refer to the biological particle that may bind to the recognition element.

Further examples of recognition elements are, e.g., nanobodies, knottins and other single-domain antibodies. Additionally or alternatively, the recognition element may comprise a (specific) protein, a (specific) peptide, an enzyme, an aptamer, and/or a nucleic acid (especially all being complementary to (at least part of) the predetermined particle). The specific protein may e.g. comprise a lectin protein such as peanut agglutinin (PNA). The recognition element may especially be a biological recognition element (for the predetermined particle) selected from the group comprising (consisting of) an antibody, a single-domain antibody, a nanobody, a knottin (an inhibitor cystine-knot), a protein, an enzyme a (poly) peptide, an aptamer and a nucleic acid. Different types of recognition elements may have affinity for a predetermined particle (a marker at the particle or a part of the particle). Yet, the affinity may vary between the different recognition elements and between the different particles. Hence, the recognition element may especially be selected and/or configured to be complementary to (to match to) (at least a part of) the predetermined particle to be sensed or detected. The recognition element may especially be selected for selectively binding with the predetermined particle. The recognition element may comprise a single-domain antibody. The recognition element may be artificially or chemically configured.

With the sensor, an interaction event of the (single) particle(s) on the electrode may be detected. In use, the sensor is configured in contact with the fluid (liquid) comprising the redox mediator, for instance in an analyzing space (see below). The electrode face is then configured in fluid contact with the analyzing space. When providing a potential difference (e.g. by functionally connecting a power supply to the electrode (face) and, e.g., a reference electrode (and/or a counter or auxiliary electrode) configured in functional connection with the fluid in the analyzing space) between the electrode face and a further location in the analyzing space (away from the electrode face and comprising the fluid, especially the liquid), (a) redox mediator in the fluid may encounter the electrode face and may exchange an electron with the electrode face (by a redox reaction). As long as the electrode face is accessible to the redox mediator (and there is a potential difference between the fluid and the electrode face), new (fresh - oxidizable or reducible) redox mediator may move (by diffusion) (from the bulk of the fluid) to the electrode face and successively (also) exchange an electron with the electrode face. It is noted that in embodiments, providing an external potential between the reference electrode and the electrode of 0 V does not mean that no potential difference exists between the electrode face and the (adjacent) fluid in the analyzing space. Therefore, providing a potential difference between the reference electrode and the electrode (face) may already inherently be done by having the (liquid) fluid comprising the redox mediator in the analyzing space (and having the electrode functionally connected to the reference electrode to provide an electrical circuit).

Hence, sensing and detection of the particle is especially based on electrochemical sensing (a presence) of the redox mediator at the electrode face. The exchange of the electron between the electrode face and the redox mediator results in a current through the electrode. The electrode thus functions as an electrochemical transducer and may also be referred to as a "sensing electrode", a "redox electrode" or a "working electrode". The reference electrode is especially configured at a distance from the (working) electrode in order to maintain a known and stable potential over the fluid (liquid). The reference electrode may in embodiments comprise an Ag/AgCl electrode. In other embodiments, the reference electrode may comprise a standard hydrogen electrode, a saturated calomel electrode, or e.g. a copper-copper(II) sulfate electrode. Yet, also other reference electrodes may be applied.

Typically, the (working) electrode is applied in combination with a reference electrode. As such, the potential of the (liquid) fluid may be controlled. Yet, it may be understood that the reference electrode may also be substituted by and/or supplemented with a further (working) electrode and/or a counter electrode without affecting the concept of the invention. Hence, in embodiments, the reference electrode may be exchanged by a further (working) electrode and/or optionally combined with a counter electrode. Herein further the term "further electrode" may be used to refer to a reference electrode as described above or a further (working) electrode not being a reference electrode. The further electrode may in embodiments comprise a counter electrode. The further electrode especially comprises an auxiliary electrode. The further electrode may (further) comprises a pseudo-reference electrode and/or a quasi-reference electrode. The further electrode may be configured in combination with a counter or auxiliary electrode.

Based on the transfer of electrons, a constant current through the electrode may be provided (or caused). This constant current is herein also referred to as "base current". Yet, if a particle (at least partly) blocks the electrode face, the particle may prevent or limit a transport of the redox mediator to the electrode face, and may thus limit the redox reaction(s) at the electrode face. Consequently, the current through the electrode changes/drops (until the particle is moved away from the electrode face again). As such, the presence of a (one) particle located at the electrode face, especially bound to the recognition element, may be sensed by measuring the current through the electrode over time. Furthermore, the presence of a further particle at the electrode (face) may provide a further drop of the current. To measure the current over time, the working electrode may be functionally coupled to a power supply and especially also the further electrode may be functionally coupled to the power supply to provide a potential difference between the further electrode (and thereby the fluid) and the working electrode. Yet, such potential difference not necessarily has to be imposed (externally by the power supply). In specific embodiments, a potential difference between the fluid and the working electrode may intrinsically be present, and especially also in such embodiment a current through the electrode may be provided when a redox mediator exchanges the electrode(s) with the electrode face. In specific embodiments, the working electrode (thus) comprises a floating electrode (i.e. an electrode on which no potential is imposed).

The potential (difference) between the working electrode and the further electrode may especially be selected based on the redox mediator (to drive the redox reaction). The potential difference may e.g. be selected positive or negative to drive an oxidation of the redox mediator at the electrode face, or to drive a reduction of the redox mediator at the electrode, respectively.

A change of the current ("a current drop") may indicate the presence of the particle (shielding the electrode face). Moreover, if the (predetermined) particle binds to the recognition element, the current may (temporarily) change over a longer period (herein also referred to as the engagement period) than if a nonspecific particle only moves over the electrode face without binding to the recognition element. Binding of the predetermined particle to the recognition element may further also be referred to as "selective binding". Hence, based on selective binding the current may at least temporarily change, especially over the engagement period. A change of the current not caused by the selective binding may be referred to as "a random change of the current" or a "random interaction of a particle at the electrode" (e.g. of a (random) particle flowing over the electrode face). Herein the change of the current may especially be explained based on a drop of the current. It will be understood that depending on the configuration, the change may also comprise a (discrete) increase in the current.

Herein, reference is made to a redox mediator. Redox mediators are known to the skilled person and are essentially molecules capable of accepting and donating an electron. Hence, the redox mediator may relate to a molecule that may be oxidized and (successively) may be reduced (or vice versa). A redox mediator may donate an electron at an anode and accept an electrode at a cathode. Herein, the redox mediator may thus (depending on the positive or negative potential at the (working) electrode face with respect to fluid, especially with respect to the further electrode) donate or accept an electron at the electrode face (from or to the electrode) thereby providing the current through the electrode. The redox mediator may especially be selected based on the net charge of the (predetermined) particle. For example, to attract a negatively charged particle on to the electrode (by electrophoretic force), the redox mediator may be selected to having a positively charged oxidized form (and vice versa). The redox mediator is especially selected for its ability to transfer electrons, to transfer charge. The redox mediator may e.g. comprise ferrocene. Ferrocene may donate an electrode resulting in the ferrocenium ion. Ferrocenium is the oxidant molecule that can be reduced to ferrocene by electrode addition and vice versa. The redox mediator may comprise a ferrocene derived redox mediator, such as ferrocenemethanol, ferrocenedimethanol, α-ferrocenylethanol. Other examples of redox mediators, e.g., comprise potassium ferricyanide, potassium ferrocyanide, or hexaammineruthenium(III) chloride. The term "redox mediator" may relate to a plurality of (different) redox mediators. The redox mediator may flow freely in the fluid and may e.g. move from the bulk of the fluid to the electrode by diffusion. The redox mediator is especially a free flowing redox mediator, especially a mobile redox mediator. The redox mediator is especially not bound/fixated to any further element.

The applicant found that the use of the redox mediator may provide a double function. The redox mediator may provide the base current as discussed above. Furthermore, the electrolysis of the redox mediator (the transfer of the electron from the electrode to the redox mediator - or vice versa) at the electrode face may generate an electrophoretic force pulling (negatively - or positively) charged or bipolar particles onto the (working) electrode. This may especially be key for detection of low-concentration of (biological) particles (with a charged surface area). This is a specific aspect of the invention: with diffusion alone, the particle will take much longer to reach the electrode face, which may prevent the detection at (clinically) relevant low concentration levels.

The electrophoretic force may apply to all charged entities in the fluid. Especially if the fluid comprises an electrolyte (as most bodily fluids do) and/or undesired particles that may provide fouling of the electrode face, it may be advantageous to provide an anti-fouling coating at the electrode face. Hence, in embodiments, the electrode (face) comprises a coating. Such coating may minimize the non-specific binding of (random) particles onto the electrode face. In embodiments, the electrode face may be chemically modified with an anti-fouling coating. The coating may be configured for repelling (undesired) proteins. The coating especially comprises a hydrophilic coating. The coating may comprise a self-assembled monolayer, or a supported lipid bilayer. The coating may comprise a protein such as bovine serum albumin (BSA). In embodiments, the coating comprises a hydrophilic polymer coating, e.g. dextran, poly(ethylene glycol) (PEG), a PEG copolymer, and a polyacrylate. In further embodiments, the coating comprises a zwitterionic polymer, for instance comprising (poly) carboxybetaine, sulfobetaine, phosphocholine and/or or hydroxy-acrylamide. In specific embodiments, the coating comprises the recognition element(s). In embodiments, the recognition element is attached to the coating. Therefore, it may also be indicated that the coating is (bio)functionalized with a specific (biological) recognition element. The coating, furthermore, is especially configured to allow an electron transfer between the redox mediator and the electrode face. The coating may comprise structural defects (pinholes) that may allow the diffusion of the redox mediator molecules through the defects towards the electrode face and/or it may allow tunneling of electrons through the defects. The coating is especially configured to not block an electron transfer between the redox mediator and the electrode face.

Hence, in a further embodiment, the electrode comprises a coating configured at the electrode face. The coating is especially configured for reducing or preventing fouling of the electrode face. The coating may further comprise the recognition element. The recognition element may be configured at the coating, and especially be attached to the coating.

When being in use, such as with the method of the invention, the electrode face is contacting the fluid (liquid) (as discussed above). Herein, the term "in fluid contact" such as in the phrases "wherein the electrode face is configured in fluid contact with the fluid" and "wherein the electrode face is configured in fluid contact with the analyzing space" is used. The term may relate to a contact between the (liquid) fluid and the electrode face directly or indirectly via the coating. The term may also relate to a contact between a gaseous fluid (in the analyzing space, especially when not being in use) and the electrode face. The fluid may be a gas or a liquid. For instance, the analyzing space may comprise a gas. However, when the electrode is being used, the fluid especially comprises a liquid (fluid) (and the redox mediator, and especially the particle if present), and the electrode face may functionally and physically contact the liquid (fluid).

The sensor is configured for sensing a predetermined particle. Moreover the sensor is especially configured for sensing a single predetermined particle, at the time (per electrode) as a discrete event. In specific embodiments, a plurality of particles being bound to the electrode may be sensed as discrete (interaction) events. The predetermined particle comprises a biological particle, such as selected from the group comprising, especially consisting of, an extracellular vesicle ("EV"), a tumor-derived extracellular vesicle ("tdEV"), a virus, a DNA-containing particle, an RNA-containing particle, a platelet, an allergen, such as peanut agglutinin (PNA), a bacterium, a peptide, a polypeptide, a protein, a lipoprotein, a hormone, a biopolymer, and an enzyme. The tumor-derived extracellular vesicle may especially comprise an extracellular vesicle presenting EpCAM (epithelial cell adhesion molecule) (marker). The tdEV may in further embodiments comprise an epidermal growth factor receptor, e.g. EGFR, ErbB-1, HER1, or HER2 that may bind to the (specific) recognition element. The tdEV may comprise a NY-ESO-1 antigen, placental alkaline phosphatase (PLAP), or Alix (apoptosis-linked gene 2-interacting protein X) that may bind to the recognition element. Hence, the recognition element may comprise an antibody for the aforementioned markers. Yet, the tdEV may also comprise another alternative marker that may be bound to a respective alternative recognition element, such as a prostate-specific antigen (PSA).

As an example of a virus, a HIV virus may be sensed and/or detected in embodiments. HIV may e.g. be selectively bound by an anti-gp120 or anti-gp41 antibody. Hence, the recognition element may in embodiments comprise such antibody. The bacterium (as an embodiment of the predetermined particle) may e.g. comprise e. coli, legionella, or salmonella. E-coli may be bound to an anti-e. coli antibody. Legionella and salmonella may be bound to an anti-legionella antibody and an anti-salmonella antibody respectively. The predetermined particle may comprise insulin, and especially the recognition element may comprise an anti-insulin antibody. In embodiments, the predetermined particle comprises testosterone and especially the recognition element comprises an anti-testosterone antibody. The hormone may further comprise hCG, adrenalin, a growth hormone, a thyroid hormone, or a luteinizing hormone. Hence, in further embodiments, the recognition element comprises an antibody for said respective hormones.

Examples of allergens (to be sensed) are e.g. specific proteins, such as milk proteins or lectins like phytohaemagglutinin, wheat germ agglutinin and peanut agglutinin.

The predetermined particle especially comprises one or more of the biological particles described herein. Moreover, the recognition element especially comprises an antibody described herein. Yet, the recognition element may additionally or alternatively comprise another recognition element that may bind to the biological particle. Hence, the recognition element may comprise an antibody for the biological particle. The recognition element, especially the antibody, is especially configured (selected) for binding the (predetermined) particle to shield at least part of the electrode face. The recognition element may be configured over the electrode face. The recognition element, especially the antibody, is in embodiments configured at the electrode face.

A bodily fluid may comprise the (biological) particle. Hence, in an embodiment, the fluid comprises a bodily fluid. Examples of such bodily fluids are blood (plasma), urine, semen (seminal fluid), vaginal secretions, cerebrospinal fluid (CSF), synovial fluid, pleural fluid (pleural lavage), pericardial fluid, peritoneal fluid, amniotic fluid, saliva, nasal fluid, otic fluid, gastric fluid, and breast milk. The fluid may comprise one or more of these bodily fluids. The fluid may e.g. comprise one or more fluids selected from the group consisting of blood, urine, saliva, sweat, seminal fluid, cerebrospinal fluid, ascites, lymph, milk, gastric acid, lacrimal fluid, and bile. The fluid may further comprise one or more of the (other) bodily fluids described herein. The fluid is especially a complex medium, especially comprising different kind of components and different kind of particles (especially including the predetermined particle).

Hence, the invention provides the sensor according to the accompanying claims for sensing a (predetermined) biological particle in a fluid, wherein the sensor comprises an electrode and an recognition element, wherein the electrode comprises an electrode face configured accessible to (i) the fluid, to (ii) the (predetermined) biological particle in the fluid, and to (iii) a redox mediator in the fluid; wherein the recognition element is configured (and/or selected) to (at least temporarily) (selectively) bind with the (predetermined) biological particle, thereby limiting access of the redox mediator to the electrode face (during binding of the (predetermined) biological particle with the recognition element). Especially, the (predetermined) biological particle is selected from the group comprising (consisting of) an extracellular vesicle (EV), a tumor-derived extracellular vesicle (tdEV), a virus, a DNA-containing particle, a particle modified with DNA, an RNA-containing particle, a particle modified with RNA, a platelet, an allergen, such as peanut agglutinin (PNA), a bacterium, a (poly)peptide, a (lipo)protein, a hormone, a biopolymer, an enzyme. The recognition element comprises a biological recognition element (such as described herein), especially an antibody, for the (predetermined) biological particle. The (biological) recognition element, especially the antibody, is in embodiments configured at the electrode face.

In a specific embodiment, the particle comprises tumor-derived extracellular vesicle (tdEV). In further embodiments, the recognition element comprises an antibody for tdEV. The antibody may in embodiments e.g. comprise an anti-EpCAM antibody, an anti-EGFR antibody, an anti-ErbB-1 antibody, an anti-HER1 antibody, and/or an anti-HER2 antibody, an anti NY-ESO-1 antibody, an anti-PLAP antibody, and/or an anti-Alix antibody.

The electrode is an electrical conductor and essentially configured for its function to provide a contact with a non-metallic part of an electric circuit. The electrode comprises an electrically conductive material. The electrically conductive material may e.g. be selected from the group consisting of copper, graphite, titanium, brass, silver, gold, platinum, and palladium. In a specific embodiment, the electrode comprises one or more of platinum and gold. In use, the fluid/liquid (functionally connected to the further electrode) is part of the electric circuit. The electrode face (of the working electrode) is especially configured for fluidly connecting to the fluid. Furthermore, the electrode face may be configured for substantially being blocked by the bound particle. Therefore, a shape of the electrode face may be configured to match a shape of the predetermined particle. The shape of the electrode face may be circular, or, e.g., rectangular. Yet, in embodiments, the shape may be irregular or elongated. The shape may especially be circular. The electrode face may further be configured concave, convex or a combination of convex and concave. The electrode face may in embodiments comprise a (circular) ring-shape (annulus) or a (rectangular) frame-shape (or rectangular "annulus") or define part of such ring-shape or frame-shape configured in a flow through channel (see below). In embodiments, the electrode face is configured flat. Also, a characteristic dimension of the electrode face may be selected to match the (predetermined) particle. A characteristic dimension of biological particles (to be sensed) may be in the range of nanometers up to hundreds of micrometers. Tumor-derived EVs may e.g. be 30 nm-1 µm, and bacteria may be 0.5-700 µm. Further bacteria may have a characteristic dimension of 300 nm - 2 µm. Further, the characteristic dimension (size) of exosomes and viruses may be 30-200 nm, and 20-400 nm respectively. Proteins may have a size in the range of 2 -25 nm, and lipoproteins in the range of 10-1000 nm and protein aggregates in the range of 20 nm-10 µm. Furthermore, platelets may only be 1 to 3 µm in size. Hence, the characteristic dimension of the electrode face may in embodiments be selected from the range of 30-1000 nm. The characteristic dimension of the electrode face is selected from the range of 30-1500 nm.. In further embodiments, the (characteristic) dimension is at least about 10 nm, like even more especially at least about 50 nm. In further embodiments, the (characteristic) dimension is in the range of at least about 100 nm.

For sensing tdEV's, the size (characteristic dimension) of the electrode face may e.g. be selected from the range of 30 nm to 1µm (depending on the type of tdEV to sense).

The electrode face may be part of a larger surface of the electrode. The term "face" with respect to the electrode is especially used to refer to a surface (optionally being part of a larger surface) of the electrode that provides the electrode function, i.e. providing the contact (of the electrode) with the fluid. The term "face" especially relates to the surface of the electrode that may be exposed to the fluid, to the particles and to the redox mediator. The term "electrode" may therefore especially relate to the electrode face in phrases like "the particles are attracted to the electrode" and "the recognition element functionally coupled to the electrode".

Hence, (based on the size of the electrode face) the electrode may especially comprise a nano-electrode. The (working) electrode may in embodiments be referred to as a "nano-disc" electrode. Moreover, the sensor, may especially comprise a nano (disc) sensor. In further embodiments, the electrode and the sensor may relate to a micro electrode/sensor. The term "characteristic dimension (of the electrode)" may especially relate to a characteristic size, such as length, a width or a diameter (of the electrode face), especially the respective size that may determine the (amount of) exposure of the electrode face to the (predetermined) particle. In embodiments, especially comprising a recessed electrode (see below), the characteristic dimension of the electrode face may be smaller than the characteristic dimension (size) of the particle, and one single particle may bind to the electrode face at the same time. The term "characteristic dimension" may especially relate to an equivalent circular diameter (of the electrode face). The equivalent circular diameter (or ECD) of an (irregularly shaped) two-dimensional shape is the diameter of a circle of equivalent area. For instance, the equivalent circular diameter of a square with side a is 2*a*SQRT(1/π).

In use, a current density at an edge of the electrode face may in embodiments be high (relative to the overall current density at the electrode face (also known as the "edge effect"). Because of that, it might still be possible for some of the redox mediator to exchange an electron with the (working) electrode even when a (large) particle is bound to the recognition element (although to a lesser extent than if no particle blocks the electrode face).

Because of the edge effect, the drop in current (when the particle binds to the recognition element) may be reduced. Therefore, in embodiments, at least part of the electrode, and especially at least part of the edge of the electrode may be enclosed/shielded by an electrically insulating base. Especially, the entire edge may be enclosed/shielded by the insulating base (wherein (only) the electrode face is still configured to be accessible to the fluid). In embodiments, the electrically insulating base and the electrode face may define a cavity. A bottom end of the cavity may be defined by the electrode face (or a coating of the electrode), and especially a wall of the cavity may be defined by the electrically insulating base. Yet, in embodiments a face of the electrically insulating base, also referred to as "insulating base face", and the electrode face may be configured at the same level (in one plane) (and thus not defining the cavity). In further embodiments, a shape of the electrode face may be selected for minimizing the edge effect. In embodiments, the electrode may be configured in an electrically insulating wall of a flow through channel. Such wall may provide the insulating face.

Hence, in embodiments, the sensor comprises an electrically insulating base enclosing at least part of the electrode, wherein the electrically insulating base comprises an insulating base face, configured coplanar to (the electrode face) or protruding from the electrode face. In further embodiments (wherein the cavity is provided), the electrode face is configured recessed in the base. Yet in other embodiments, the electrode face is configured protruding from the base.

In specific embodiments, the base comprises a (insulating) substrate layer supporting the electrode, and a (insulating) passivation layer covering the substrate layer, wherein the passivation layer encloses the electrode (with the electrode face configured to be accessible to the fluid). Hence, in a further embodiment the base comprises a substrate layer and a passivation layer, wherein the substrate layer comprises a substrate layer face, wherein the passivation layer is configured covering at least part of the substrate layer face, wherein the electrode face is configured protruding from the substrate layer face, and wherein the passivation layer at least partly encloses the electrode (wherein the electrode face is configured to be accessible to the fluid). In such embodiment, the passivation layer and the electrode face may define the cavity, and especially the insulating base face comprises a face of the passivation layer. Such embodiment of the sensor or of the device comprising the sensor may easily be made by (micro/nano) fabrication methods. The fabrication method may e.g. comprise lithography.

Herein, the terms "electrode" and "electrode face" in phrases like "the electrode face of the electrode" and the like may relate to more than one electrode (each having a respective electrode face). Furthermore, the term "particle" may relate to a plurality of (different) particles. The term may refer to a predetermined particle, especially being a particle that (selectively) binds with the recognition element. The term may also refer to a (non-specific) (random) particle that does not bind to the recognition element. The term may also relate to different predetermined particles that may (each) bind to (different) electrodes comprising different recognition elements. The particle may especially be present (or absent) in a complex medium.

The sensor comprises an array of electrodes. The array comprises a plurality of electrodes (each comprising a respective recognition element). The array may in embodiments, e.g., comprise two, three, four, six, nine, sixteen, or twenty-five electrodes. In embodiments, the array comprises even more, or another number of electrodes. The array may be a 1D array, especially wherein all electrodes are arranged in line. Yet, the electrodes may in further embodiments be arranged in a plane of the array. The array may in embodiments be a 2D array. In further embodiments, the array of electrodes may comprise an array of arrays of electrodes. Each electrode is functionally coupled to a (one or more) respective recognition element(s). Further, each electrode face comprises at least one respective recognition element. In embodiments each of the electrodes (of the array) is configured for sensing a (respective) predetermined particle (independently from another one of the electrodes) (or no predetermined particle). In embodiments, substantially all, such as at least 90% or 95%, of the electrodes may sense the same (type of) particle. Substantially all electrodes (of the array) may e.g. be configured for sensing tdEVs. Yet, in further embodiments, the recognition element of at least one of the electrode faces is configured to bind another predetermined particle than the recognition element of other electrodes of the array. Different particles especially have different physical characteristics.

Hence, in embodiments the sensor comprises an array of electrodes, wherein each of the electrodes is configured for sensing a (respective) predetermined particle (independently from another one of the electrodes). The electrodes are especially configured for individually sensing a predetermined particle. The sensor may be configured for sensing a predetermined number of different predetermined particles. The predetermined number of different particles may be equal to or less than a total number of electrodes of the array. The term "array of electrodes" may in embodiments refer to a plurality of (different) arrays of electrodes. The electrodes of the array of electrodes are especially individually accessible to the (respective) predetermined particle(s) (and the redox mediator). Each of the electrodes of the array of electrodes is configured for detecting a single/individual (determined) particle.

In yet further specific embodiments, an electrode may also be used to detect different predetermined particles by using different types of recognition elements. Such different particles may lead to different signals, such as different signal height, which may in an individual level be monitored.

The electrode (a single electrode or an electrode of an array of electrodes) may be characterized by an electrode characteristic(s). In further embodiments, (optionally) at least one of the electrodes of the array has the electrode characteristic being different from the electrode characteristic of the other electrodes of the array. Such electrode characteristic(s) may e.g. be one or more of the characteristic dimension of the electrode face, the coating of the electrode, the recognition element(s) at the electrode face, and the electrically conductive material. In use, further electrode characteristics may comprise the voltage (potential) of the electrode face and a flow rate of the fluid along the electrode.

In use, the sensor is preferably configured in fluid (liquid) connection with the fluid (liquid). Therefore, the sensor may be part of a device configured to host the fluid (liquid). Hence, in a further aspect, the invention provides a device for analyzing a fluid, wherein the device comprises an analyzing space comprising the sensor described herein. The sensor especially comprises the electrode wherein the electrode face is configured in fluid contact with the analyzing space.

It will be understood that the term "the electrode" and "electrode face", and the like may refer to a sensor comprising one (working) electrode, or a plurality of (different) (working) electrodes, as well as to a sensor comprising an array of (working) electrodes (or a plurality of arrays). As such, for a sensor comprising an array of electrodes or more than one electrode, each respective electrode face is especially configured in fluid contact with the analyzing space. Hence in use, the analyzing space is provided with the fluid (liquid), and the one or more electrode faces are (in functional and) in physical contact with the fluid (liquid). Furthermore, during use, the liquid (fluid) is also especially in functional contact with a further electrode (e.g. reference electrode) configured in the device and/or configured in fluid contact with the liquid (fluid) in the device (and in functional contact with the electrode face(s) for providing the electrical circuit(s)). Hence, in embodiments, the device further comprises a further electrode (a reference electrode) (and/or optionally a counter electrode) configured for fluidly connecting with the analyzing space.

The analyzing space may comprise an analyzing chamber configured for holding the fluid (liquid). In embodiments, the sensor may be enclosed by the chamber. Yet, in further embodiments, the sensor, especially the electrode may be arranged in a wall of the chamber. In specific embodiments, the device comprises a channel and especially the chamber may be configured in the channel. The chamber may be arranged at a terminal end of the channel. Yet, in further embodiments, the channel comprises a flow through channel (and the chamber is a flow through chamber). The term "flow through" in expressions like "flow through channel" especially refers to a device such as a channel wherein a fluid may enter the device at a first location (or end) and exit the device at a further location (or end), different from the first location.

A flow through channel may thus refer to a channel in which the fluid enters at a first extreme of the channel along an axis of the channel and leaves at a second location, such as a second extreme, of the channel along the channel axis. Additionally or alternatively, the fluid may leave the channel via e.g. an opening (through hole) or a pore configured in the wall of the channel. The openings may be configured in a part of the wall being configured substantially coaxially with the channel axis. The fluid may leave the channel in such embodiment for instance in a direction perpendicular to the axis of the channel. In further embodiments a part of the channel wall may be arranged inside the channel (substantially perpendicular to the channel axis) and as such defining the chamber in the channel especially upstream of the part of the channel wall arranged inside the channel. Additionally or alternatively, said part of the wall may comprise at least part of the openings and especially (at least part of) the fluid may leave the chamber in a direction parallel to the axis of the channel. Hence, also such embodiment comprises a flow through channel. The pore(s) or opening(s) may especially be configured for providing a fluid connection through the channel wall from inside the channel (from the chamber) to external of the channel (the chamber).

In further embodiments the chamber is configured at the terminal end of the channel wherein a fluid may only enter the chamber and exit the chamber at one location. Such channel may thus not be a flow through channel. The plurality of electrodes may in embodiments be arranged at a part of the wall. The electrodes may further be arranged in the wall. The electrode(s), e.g., may be configured in (a) pore(s) or opening(s) configured in the wall. The electrodes, especially the array of electrodes, may be arranged (in a direction) along the axis of the channel. In further embodiments, at least a part of the electrodes is arranged in a plane (or a plurality of planes) perpendicular to the channel axis. The electrodes, e.g., may be evenly distributed coaxially around the channel axis. Yet, in alternative embodiments, the channel wall comprises pores distributed over the channel wall. The pores may be (spatially) distributed evenly over the channel wall. The pores may especially be distributed randomly (and optionally also evenly) over the channel wall. In a specific embodiment, in substantially each pore, an electrode is configured. Hence, in embodiments, (also) the channel wall defines (a part of) the analyzing space. The fluid (liquid) may flow through the channel (wall), and especially a particle may be bound to a recognition element of an electrode in the channel wall. In embodiments, the electrode in the channel wall or pore may comprise at least part (such as 180 ° or 300 °) of a ring-shape electrode. In further embodiments the electrode may comprise a complete (360°) ring-shape. Such electrodes (with at least part of a ring-shape) may also be called a "flow through electrode".

Hence, in further embodiments, the device comprises a channel with a channel wall, wherein the channel (wall) defines the analyzing space, and wherein the channel wall comprises the electrode (and the electrode face(s) is (are) configured accessible to the (liquid) fluid in the analyzing space when the (liquid) fluid is present in the analyzing space). In specific embodiments of a flow through channel, the analyzing space may further be defined by one or two valves, especially check valves, configured in the channel and enclosing the analyzing space. In embodiments, one check valve and the channel wall (comprising pores and/or openings) may define the analyzing space. In further embodiments, two valves (together with the channel wall) define the analyzing space Especially, the valve(s) comprise(s) electrical insulating material (wherein the further electrode is configured in the analyzing space). In other embodiments, especially wherein the further electrode is configured out of the analyzing space, the valve may comprise electrically conductive material.

In yet a further aspect, the invention provides a system for analyzing a fluid ("system") comprising the device described herein. The system further comprises a further electrode (especially a reference electrode) (and optionally a counter electrode), wherein the further electrode (and the optional counter electrode) is configured to functionally connect to the fluid in the analyzing space (during operation of the system). Especially, the further electrode and the electrode (face) are configured to provide an electrical circuit when the fluid (liquid) fluidly connects the further electrode with the electrode face. To further provide the circuit, the electrode and the further electrode may be (functionally) connected to each other. In embodiments the system comprises a power supply functionally connecting the electrode face(s) with the further electrode. Functionally connecting electrodes may comprises (conductively) coupling the electrodes directly and/or via a power supply. The terms "further electrode" "reference electrode" and "counter electrode" may in embodiments refer to a plurality of further electrodes and/or reference electrodes, and/or counter electrodes. The counter electrode may especially be functionally connected with the further electrode, especially with the reference electrode.

The system further comprises a control system configured to execute a measuring routing (in a control mode). The fluid may be analyzed during the measuring routine. In embodiments, the measuring routine may comprise providing a potential difference between the further electrode and the (one or more) electrode (face(s)) and measuring the electric current through the electrode (when the fluid is present in the analyzing space and when the further electrode and the electrode face(s) are functionally coupled (especially via the power supply)). The measuring of the current through the electrode especially relates to measuring the electric current through the electrical circuit (provided during operation of the system). The current may be measured using an ammeter. Yet, combined systems are commonly used for providing a potential difference and measuring a current. Herein, the system and the method may be explained using a power supply and an ammeter. It will be understood that other (dedicated) systems known in the art may be used in the method and/or configured in the device/system of the invention. Furthermore, as discussed above, the power supply not necessarily has to be used for providing the potential difference between the electrodes.

Hence, the system further comprises a control system, wherein the control system is configured to execute a measuring routine comprising measuring during an analyzing period an electric current through the electrode caused by a potential difference between the further electrode (reference electrode) and the electrode (face), wherein the system, further comprises an electric current measuring device configured to measure the electric current through the electrode. In further embodiments, the system further comprises an electric power supply configured for providing the potential difference between the further electrode and the electrode (face). The electric current is especially measured when the fluid (liquid) is provided in the analyzing space. The system may in embodiments comprise more than one power supply configured for providing the potential difference between more than one electrode and the further electrode(s).

In specific embodiments, the channel comprises the flow through channel. As such (at least a fraction of) the fluid may be provided to the channel (by a fluid transport device), especially to (in) the analyzing space, maintained in the analyzing space and removed from the analyzing space again, especially by flowing the fluid through the channel. By repeatedly providing fluid to the channel, successively fractions of the fluid may be analyzed in the analyzing space. The system, especially the fluid transport device, may e.g. be configured to provide the fluid in pulses or interruptedly to the channel. In specific embodiments, the system may be configured for providing a continuous flow of the fluid to the channel. Additionally or alternatively, the system may be configured for providing discrete volumes of the (liquid) fluid to the channel wherein the discrete volumes (of the liquid) are separated from each other by a separation fluid (see below). The system may be configured for providing the discrete volumes (of the liquid) interspaced with the separation fluid to the channel (and sequentially into the analyzing space). The system thus especially further comprises a transport device. The control system may be configured for controlling the transport device (to provide the fluid to the analyzing zone (as described above)).

In embodiments, (especially wherein the device comprises the channel) the system further comprises a fluid transport device, wherein the fluid transport device is functionally connected to the analyzing space, especially to the channel, wherein the fluid transport device is configured to (i) provide the fluid to the analyzing space and to (ii) remove the fluid from the analyzing space after (and/or during) maintaining the fluid in the analyzing space during the analyzing period (in the measuring routine).

In specific embodiments, the system is configured for providing a series of (discrete) volumes of (the) fluid to the channel, wherein the (discrete) volumes of (the) fluid are separated from each other by a separation fluid, and wherein the system is further configured to successively execute the measuring routine for each volume of (the) fluid (in the analyzing space) (by flowing the series of volumes of (the) fluid through the analyzing space), wherein successively (i) each volume of the series of volumes of (the) fluid is provided to the analyzing space and (successively) (ii) removed from the analyzing space after (and/or during) maintaining each volume in the analyzing space during the analyzing period (and measuring each volume during the analyzing period (by measuring an electric current through the electrode caused by a potential difference between the further electrode and the electrode (face))).

The separation fluid may comprise a gas or (also) e.g. a liquid, such as an aqueous liquid, especially water. The separation fluid may further comprise a compound to clean the analyzing space. The separation fluid may in embodiments be selected to detach any bound particle from the recognition element(s).

The terms "the fluid" (and "the liquid") in phrases like "providing a series of volumes of the fluid (the liquid)" and "flowing the series of volumes of the fluid (liquid) through the analyzing space" may in embodiments relate to a plurality of (different) fluids (liquids). The above given embodiment may e.g. be configured for providing discrete volumes of different liquids (fluids) to the channel. By using such embodiment (in the method of the invention) also a plurality of liquids (fluids) may be analyzed (in one or more runs or series).

In yet a further aspect, the invention provides a method for analyzing (a) fluid ((a) liquid). The method comprises providing the system described herein (comprising a channel and a fluid transport device) and functionally connecting the further electrode to the electrode face, especially via the electric power supply. The method especially further comprises: during a measuring stage: (i) providing the fluid (liquid) comprising a redox mediator to the analyzing space, (ii) executing a measuring routine during an analyzing period, and especially (iii) removing the fluid (liquid) from the analyzing space again. The measuring routine comprises: measuring an electric current through the respective electrode as a function of time (caused by a potential difference between the further electrode and the electrode faces) (thereby providing measured electric current data as a function of time).

In embodiments, the electric power supply is connected to the further electrode and the electrode face. In embodiments, the measuring routine further comprises providing the potential difference between the further electrode and the electrode faces by means of the electric power supply.

In embodiments, the fluid (liquid) is maintained in the analyzing space during the analyzing period. In specific embodiments, continuously, the fluid (liquid) (comprising the redox mediator) may be provided to the analyzing space (flown through the analyzing space), wherein (continuously) fluid (liquid) is forced out of the analyzing space again (e.g. via the openings / pores in the channel wall).

As discussed above, for embodiments comprising a plurality of electrodes and/or an array of electrodes, the further electrode is functionally connected to one or more, especially to each electrode, especially via the electric power supply. Furthermore, a potential difference may (externally) be provided between the further electrode(s) and one or more of the electrode faces and a respective electric current through the one or more electrodes may be measured as a function of time. In embodiments, a different potential difference may be provided by the electric power supply between a first electrode (electrode face) (of the array of electrodes) and the further electrode than between another electrode (of the array of electrodes) and the reference electrode (by the same or by another power supply). In further embodiments, the same potential difference is provided between each of the electrodes (of the array of electrodes) and the further electrode.

In embodiments the fluid (liquid) comprises a bodily fluid, especially a fluid (liquid) selected from the group consisting of blood, urine, saliva, sweat, seminal fluid, cerebrospinal fluid, ascites, lymph, milk, gastric acid, lacrimal fluid, and bile.

The method may further comprise an analyzing stage, wherein the measured electric current data as a function of time is analyzed, especially statistically analyzed. By means of the (statistical) data analysis, a selective binding of the particle to the recognition element may be differentiated from a random interaction of a particle at the electrode. Especially, a statistically relevant minimal period of the current change (the engagement period) that indicates the selective binding of the particle may be determined in the analyzing stage. Furthermore, in the analyzing stage a change in the electric current, especially a difference relative to the base current ("a current drop"), during a (random and/or specific) interaction of a particle with the electrode face may be determined. Especially, a total number of (such) changes (current drops) during the analyzing period may be determined in the analyzing stage.

In embodiments, (the method for) analyzing the fluid (liquid) comprises determining (concluding/establishing) a presence of a particle (or a plurality of particles), wherein the presence of the particle(s) is determined based on a change in the measured electric current (value) as a function of time, especially by a (current) drop in the measured current during a determined time period. The time period may especially be determined based on the statistical analysis. A change in the measured current may indicate that the electrode face is blocked by a particle or any particulate material which does not bind to the recognition element. Hence, such change may only be observed a fraction of the analyzing period. The current drop may also be measured/observed during a substantial part of the analyzing period, especially during a (pre)determined length of the analyzing period (especially the statistical relevant minimal period). The (extended) current drop may e.g. be measured/observed during at least 1% of the analyzing period, such as at least 5% or at least 10% of the analyzing period, especially during at least 20% of the analyzing period. The predetermined length of the analyzing period especially comprises the engagement period. The extended current drop may especially be observed during engagement periods described herein. The (pre) determined length of the analyzing period (wherein the change of current is observed), may especially be at least 25 milliseconds, especially at least 50 milliseconds and especially equal to or less than 10,000 milliseconds, such as equal to or less than 5000 milliseconds, especially equal to or less than 2500 milliseconds). Such extended current drop may indicate the presence of a predetermined particle that is bound to the recognition element (being configured to selectively bind to the predetermined particle. It will be understood that a plurality of changes may be superimposed on each other when a plurality of particles (successively) bind (and/or release) to (and from) the electrode.

Hence in embodiments, the method further comprises an analyzing stage wherein the measured electric current data as a function of time is analyzed. The analyzing stage may comprise analyzing the fluid. In embodiments, the analyzing stage comprises determining the presence of a (predetermined) particle (in the fluid), wherein the presence of the (predetermined) particle is determined (concluded) based on a change in the measured electric current (value) as a function of time, especially based on a (current) drop in the measured current during a determined time period.

Hence, analyzing the fluid (liquid) further comprises determining a presence of a predetermined particle in the fluid (liquid). The presence of the predetermined particle may especially be determined based on a minimal duration of a (pre) determined change in the measured electric current as a function of time, especially based on a change during a (pre) determined length of the analyzing period. The minimal duration (the determined length) is especially a period equal to or larger than the statistically relevant minimal period (the engagement period).

Moreover, a number of (such) current drops (determined changes) measured during the analyzing period may relate to a concentration of the predetermined particle in the fluid. A number of current drops may be measured using an array of electrodes or a plurality of electrodes configured to selectively bind the predetermined particle. In a further embodiment, analyzing the fluid further comprises determining a concentration of the predetermined particle in the fluid, wherein the concentration of the predetermined particle is determined based on a number of determined changes over (lasting) at least the minimal duration in the measured current as a function of time, especially relative to the analyzing period.

Furthermore, a size (or a characteristic dimension) of the (predetermined) particle (relative to a size (characteristic dimension) of the electrode face) may affect the accessibility of electrode face for the redox mediator. If the predetermined particle only blocks the electrode face for a small part of the face, the drop in current, i.e. the amplitude of the current drop, may be much less than if the predetermined particle almost completely limits access of the redox mediator to the electrode face. Such partly blocking may (also) provide a discrete change in the measured current over time (signal). Hence, in a further embodiment, analyzing the fluid (liquid) further comprises determining a size of the particle in the fluid (liquid), wherein the size of the particle is determined based on a magnitude (amplitude) of the change of the current as a function of time, especially wherein a larger measured magnitude of the current change indicates a larger the size of the particle. The terms "current drop" and "change of the current" especially relate to the difference (in value) between the base current and the (temporarily) measured current. In alternative embodiments, the size (characteristic dimension) of the electrode (face) is selected based on the size of the particle to be determined (see above). The predetermined particle may especially block the face (substantially) entirely. The size of the face of the electrode may be selected to allow single particle detection. The term single particle detection especially relates to a detection of a single/individual particle being bound at the electrode. The term may relate to a discrete change in the measured current over time. In embodiments, binding of one individual particle with a recognition element may block access of another particle to the electrode face. In further embodiments, more than one particle may bind at the electrode and binding of a further particle (and/or release of a bound particle) may show a (further) discrete change in the electric current through the electrode.

The method may especially comprise chronoamperometry. Chronoamperometry may especially allow following (detecting) individual (interaction) events in time, especially based on a changing access of the redox mediator to the electrode face (as a result of a particle being bound to the electrode or being released from the electrode)

In a specific embodiment, the method comprises a method for analyzing a series of volumes of fluid (liquid). The method may especially comprise providing a series of volumes of fluid (liquid) comprising the redox mediator to the channel, wherein the volumes of the fluid (liquid) are separated from each other by a separation fluid. The measuring stage may then especially comprise: flowing the series of (interspaced) volumes of fluid (liquid) comprising the redox mediator through the analyzing space, thereby sequentially (i) providing one of the volumes of the series of volumes to the analyzing space, (ii) executing the measuring routine during the analyzing period (wherein the respective volume of fluid (liquid) is maintained in the analyzing space during the analyzing period), and (iii) removing the respective volume of fluid (liquid) from the analyzing space again, (thereby providing the separation fluid to the analyzing space). Especially, this way the respective volumes of fluid (liquid) may be analyzed sequentially.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figs 1 and 2 schematically depict some aspects of the sensor;
Figs 3 and 5 schematically depicts some aspects of the device and system;
Fig. 4 schematically depicts some further aspects of the invention.

The schematic drawings are not necessarily to scale.

### DETAILED DESCRIPTION OF THE EMBODIMENTS

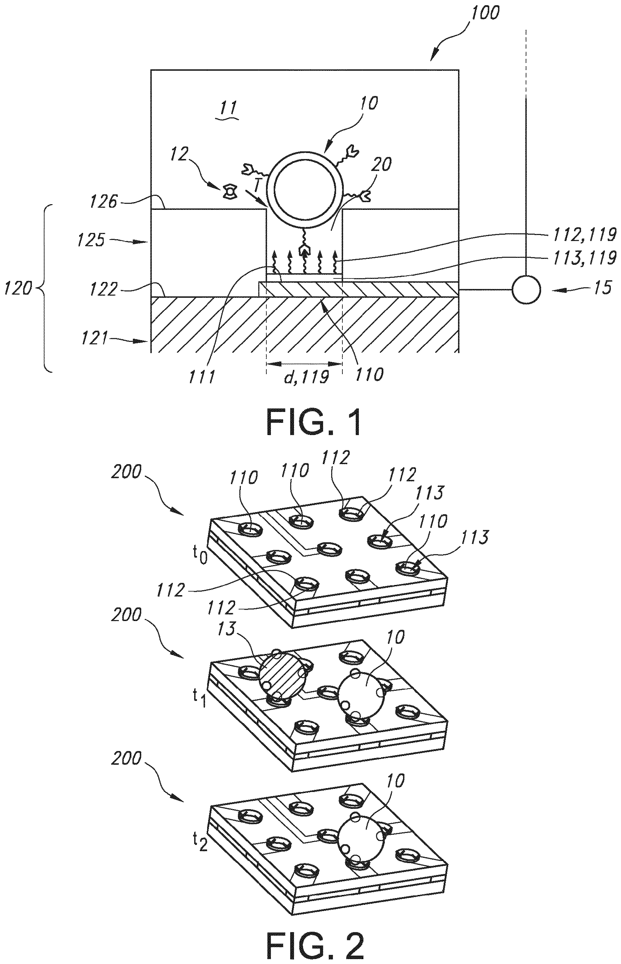

Fig. 1 schematically depicts aspects of the sensor 100 for sensing a predetermined particle 10 in a fluid 11. The sensor 100 comprises an electrode 110 and a recognition element 112. The electrode face 111 is configured accessible to the fluid 11, to the predetermined particle 10 in the fluid 11, and to a redox mediator 12 in the fluid 11. The recognition element 112 may especially (at least temporarily) selectively bind with the predetermined particle 10 thereby limiting access of a transport T of the redox mediator 12 to the electrode face 111 as is indicated in the figure. Hence, the figure depicts a status during binding of the particle 10 with the recognition element 12, herein also indicated as the engagement period P.

The fluid 11 may e.g. comprise a bodily fluid, such as of blood, urine, saliva, sweat, seminal fluid, cerebrospinal fluid, ascites, lymph, milk, gastric acid, lacrimal fluid, and bile. Herein, the invention is especially explained based on a biological particle, especially tdEV, in a bodily fluid. Yet, the fluid 11 not necessarily is a bodily fluid, but may e.g. in embodiments comprise (environmental) water. The predetermined particle 10 may e.g. comprise a biological particle 10 such as selected from the group consisting of a (tumor-derived) extracellular vesicle ((td)EV), a virus, a DNA-containing particle, an RNA-containing particle, a (poly)peptide, a protein (including a lipoprotein), and an enzyme. The biological particle 10 may in further embodiments comprise a particle modified with DNA (a DNA-functionalized particle) or a particle modified with RNA (an RNA-functionalized particle). In yet further embodiments, the biological particle 10 may comprise a particle selected from the group consisting of a platelet, an allergen, a bacterium, a hormone, and a biopolymer. The recognition element 112 may thus comprise a biological recognition element 112 for the predetermined particle 10 and may for instance be one or more of an antibody, a single-domain antibody, a nanobody, a knottin, a peptide, an aptamer or a nucleic acid. Herein terms like "a biological recognition element for the biological particle", etc. are used indicating that the particle 10 has a high (chemical) affinity for biological recognition element 112. At least a part of the particle 10 (herein said part may also be indicated as "marker") may especially bind to recognition element 112 as is schematically indicated in Fig. 1 by means of matching shapes of the particle 10 and the recognition element 112.

The electrode face 111 comprises the recognition element 112. In the depicted embodiment is the recognition element 112 configured at the electrode face 111. In other embodiments, the element 112 may be configured at a location near the electrode face 111 allowing to block the electrode face 111 for the redox mediator 12.

The sensor 100 especially comprises an electrically insulating base 120 enclosing at least part of the electrode 110. To minimize an edge effect, the insulating base face 126 of the electrically insulating base 120 may especially be configured parallel to the electrode face 111. In specific embodiments, e.g. depicted in Fig. 1, the insulating base face 126 is configured protruding from the electrode face 111, and a cavity 20 is defined by the electrode face 111 and (the passivation layer 125 of) the insulating base 120. This may also be indicated as a recessed electrode face 111. In the depicted embodiment, the electrode face 111 is configured recessed in the base 120 and the passivation layer 125 encloses the electrode 110. The base 120 further comprises a substrate layer 121 comprising a substrate layer face 122. The passivation layer 125 is configured covering at least part of the substrate layer face 122. Furthermore, the electrode face 111 protrudes from the substrate layer face 122.

The electrode 110 of the depicted embodiment comprises a coating 113 at the electrode face 111 comprising a plurality of recognition elements 112. The coating 113 is not necessarily made of a conductive material but is configured to allow an electron transfer between the electrode face 111 and the redox mediator 12, e.g., because of channels or pores in the coating 113. The coating 113 may further be configured for reducing or preventing fouling of the electrode face 111.

Fig. 1 further depicts a part of the sensor 100 comprising an array 200 of electrodes 110, such as depicted in Fig. 2. In embodiments, all electrodes 110 of the array 200 may be the same. Yet, in further embodiments at least one of the electrodes 110 of the array 200 has an electrode characteristic 119 being different from the electrode characteristic 119 of the other electrodes 110 of the array 200. Examples of the electrode characteristic 119 are depicted and may e.g. be the dimension d, especially the equivalent diameter, of the electrode face 111, the (type of) coating 113, the (type of) recognition element 112, and the conductive material of the electrode 110.

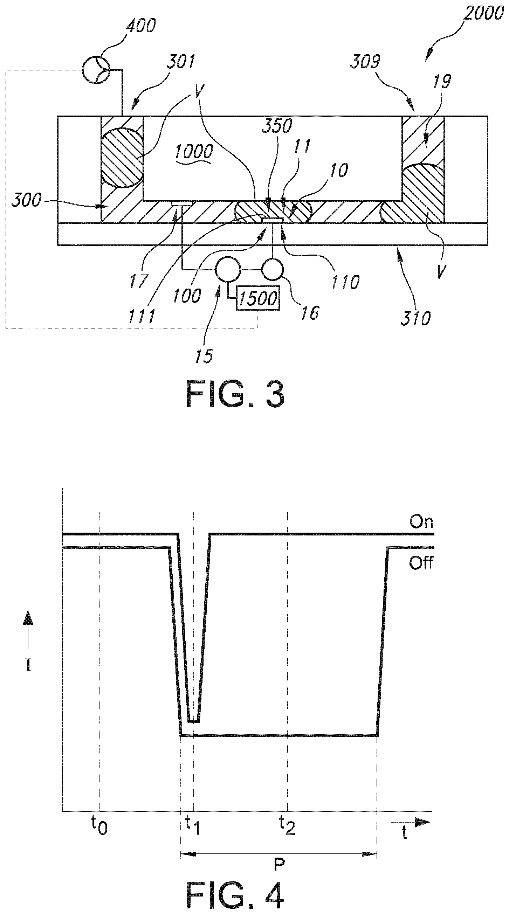

Fig. 2, especially in combination with Fig. 4, further depicts the difference between selectively binding of a predetermined particle 10 to the recognition element 12 thereby limiting the transfer T of the redox mediator 12 and a determination of another (random) particle 13 that does not bind to the recognition element 12, but still may block the electrode face 111 for the redox mediator 12 for a small period of time. At the top, at t=t₀, no particle is present near the electrodes 110 of the array 200. At t=t₁ a predetermined particle 10, depicted by the unshaded particle 10, is bound to the recognition element 112 of one of the sensors 100, and a random (not predetermined) particle 13, depicted as the shaded (hatched) particle 13, is located in front of another electrode 110 of the array 200. Because of the presence of both particles 10, 13, a transport T of the redox mediator 12 is limited to the respective electrode 110. Hence, when measuring the current I over time t (see Fig. 4), the current I may drop at t=t₁, relative to the current I at t=t₀. Yet a little later, at t=t₂, the random particle 13 is diffused away again, but the predetermined particle 10 is still located at the electrode 110 because it is bound to the recognition element 12. Because of this difference, during measuring the electric current I through the respective electrodes 110 as a function of time t for the electrode 110 with the random particle 13 (top line in Fig. 4) only a current drop is observed over a small period, whereas for the electrode 110 with the predetermined particle 10 (bottom line in Fig. 4) a current drop over a much longer period, especially over the engagement period P is observed. The engagement period P is not necessarily a fixed period and may depend on many parameters as discussed before. Therefore, statistical analysis may help discriminating between a change in current I as a result of a bound particle 10 or e.g. as a result of a particle 13 that moves over the electrode face 111. Based on data analysis e.g. a minimal relevant period (a minimal engagement period P) may be determined that may be indicative for the bound (predetermined) particle 10.

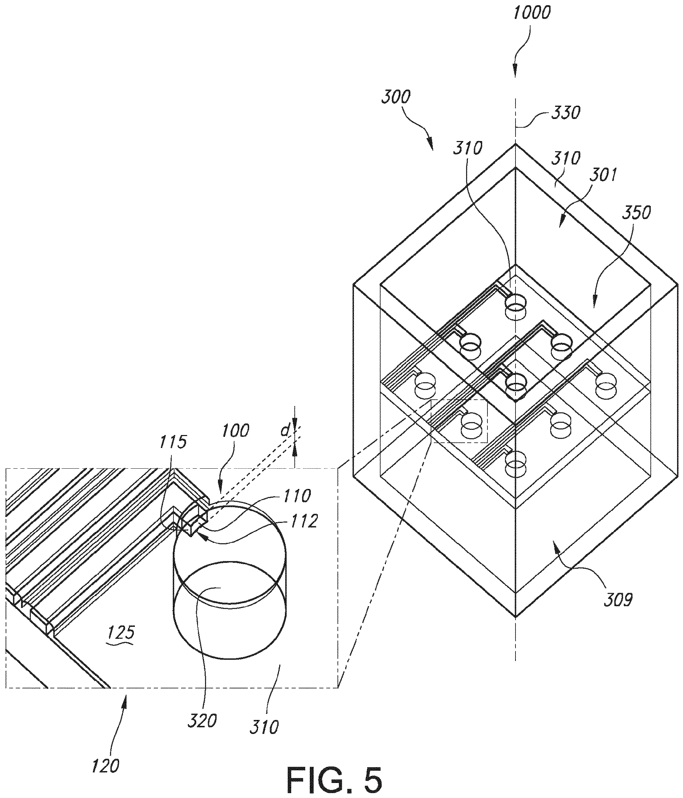

The sensor 100 may be part of the device 1000 for analyzing a fluid 11 as depicted in Fig. 3. The device 1000 comprises an analyzing space 350 comprising the sensor 100, and the electrode face 111 is in fluid contact with the analyzing space 350. In the device 1000 in Fig. 3, a channel 300 defines the analyzing space 350 and the channel wall 310 of the channel 300 comprises the electrode 110. The channel 300 of depicted embodiment may be named a flow through channel, configured for providing the fluid 11 at a first end 301 of the channel 300 and having the fluid 11 exit at another end 309 of the channel 300. In Fig. 5 another type of flow through channel is depicted. The channel 300 comprises openings (through holes) 320 or, e.g., pores 320 configured in the wall 310 of the channel 300. It is noted in embodiments, the openings 320 may be configured in the wall 310 or the part of the wall 310 configured parallel to the longitudinal axis of the channel 300. Additionally or alternatively, the openings 320 may be configured in a part of the wall 310 of the channel 300, especially arranged perpendicular or traverse to the longitudinal channel axis 1000, as is depicted in Fig. 5. The sensor 100 may be configured in the opening(s) 320. In such embodiment, the fluid 11 including the particle 10 may flow through the opening 320, wherein the predetermined particle 10 may be facilitated to encounter the electrode 110 with the recognition element 112. Using such embodiment, it may be advantageous to continuously provide and remove fluid 11 to the channel 300, without maintaining the fluid in the analyzing space 350 during a discrete analyzing period. In further embodiments, the analyzing space 350 may also be configured at a closed end of a channel 300, wherein the fluid 11 may have to enter and leave the channel at the same location. In such embodiment, the characteristic dimension d may especially be the length (or the width) of the electrode 100, especially defining the exposure of the electrode 100 to the particle 10. The electrode 100 in the embodiment is especially rectangular. In further embodiments (not shown), the electrode 100 may be (a section of) a ring-shaped (cylindrical) (comprising an annulus), e.g. (a section of) a cylinder surrounding the opening 320. Such electrode may herein also be referred to as flow-through electrode.

Fig. 3 also depicts aspects of the system 2000 for analyzing the fluid 11. The system 2000 comprises the device 1000, a further electrode 17 functionally connected to electrode 110 (face 111), and an electric current measuring device 16 configured to measure the electric current I through the electrode 110, caused by a potential difference between the fluid 11 and the electrode face 111 / electrode 110. The embodiment further comprises an electric power supply 15, configured for providing the potential difference between the further electrode 17 and the electrode face 111. The further electrode 17 is functionally connected to the fluid 11 in the analyzing space 350. The system 2000 further comprises a control system 1500 configured to execute the measuring routine (at least comprising measuring during an analyzing period an electric current I through the electrode 110 caused by a potential difference provided between the further electrode 17 and the electrode face 111). The control system 1500 is especially functionally connected to the electric current measuring device 16 and may further be connected to the electric power supply 15. For further automation, the control system 1500 may also be functionally connected to a transport device 400 that is functionally coupled to the channel 300 and that is especially configured to provide the fluid 11 to the analyzing space 350 and to remove the fluid 11 again from the analyzing space 350 (after having maintained the fluid 11 in the analyzing space 350 during the analyzing period). In the embodiment of Fig. 3, the system 2000 comprises the fluid transport device 400.

In embodiments, the system 2000 is configured for measuring only one sample of the fluid 11. Yet, in specific embodiments, as depicted in the figure, the system 2000 is configured for providing a series of volumes V of the fluid 11 to the channel 300, wherein the volumes V are separated from each other by a separation fluid 19. The system 2000 is further especially configured to successively execute the measuring routine for each volume V of the fluid 11 by flowing the series of volumes V of the fluid 11 through the analyzing space 350. As such, successively (i) each volume V is provided to the analyzing space 350 and again removed from the analyzing space 350 after having been maintained in the analyzing space 350 during the analyzing period. Hence, in such embodiment, the control system 1500 may be functionally connected to the fluid transport device 400, and the control system 1500 may especially be configured to sequentially execute the measuring routine for each volume V of the series of volumes V of the fluid 11.

The method of the invention may be applied in the system 2000 having the electric power supply 15 functionally connected to the further electrode 17 and the electrode face 111. Yet, the method may also be applied without the power supply 15. In the method, the fluid 11 comprising a redox mediator 12 is provided to the analyzing space 350. Successively, while maintaining the fluid 11 in the analyzing space 350, a measuring routine is executed during an analyzing period. And next, the fluid 11 is removed again from the analyzing space 350. In embodiments, a potential difference between the fluid 11 and the electrode face 111 may already intrinsically be present. However, during the measuring routine, also an external potential difference may be provided between the further electrode 17 and the electrode face 111 by the power supply 15. During the measuring routine, an electric current I through the electrode 110 may be measured as a function of time t. The results (based on a system 2000 comprising two electrodes 110) may for instance be like the graphs depicted in Fig. 4. That graph shows the presence of two particles 10, depicted by a change in the measured electric current I (a current drop) as a function of time t for both electrodes 110. Based on the duration of the two graphs it may be concluded that a predetermined particle 10 was present. Furthermore, in embodiments a concentration of the predetermined particle 10 in the fluid 11 may be determined based on a number of determined current drops lasting at least during a minimal relevant duration. In embodiments, multiple particles may be bound to the electrode, which may be shown by discrete steps (drops when binding a particle and increases when releasing a particle) in such graphs. Based on the amplitude of the change in the current I, in embodiments (also) a size of the particle 10 in the fluid 11 may be determined. The change in the current I especially depicts discrete (interaction) events, each event depicting the presence of a each (single) particle. In embodiments of the method, sequentially a series of volumes V of the fluid is provided to the analyzing space and analyzed.

Hence, the invention is especially based on a time-resolved electrochemical detection of discrete interaction events of the particle(s) on the electrode. In embodiments, the invention relates to functionalized electrodes.

An amperometric detection method may be used comprising electrolyzing a redox mediator (e.g., ferrocene) on the (nano)electrode, giving a constant base current. Particles (e.g., tdEVs) that are not electroactive, and are immobilized on the nanoelectrode surface, may block a mass transfer of the redox mediator onto the electrode, especially resulting in a decrease of (the amplitude of) this base current. The electrolysis of the redox mediator on the electrode may jointly generate an electrophoretic force pulling (especially negatively) charged particles onto the electrode, which may allow a low-concentration analyte detection. However, this electrophoretic force essentially applies to all charged entities in the solution. Consequently, a highly effective anti-fouling layer 113 may in embodiments be configured to minimize non-specific binding of (random) particles 13 onto the electrode surface 111. To this aim, the surface 111 of the electrodes 110 may be chemically modified with an anti-fouling layer (e.g., zwitterionic or poly(ethylene glycol) layers) to avoid non-specific binding. The anti-fouling layer may further e.g. be functionalized with tdEV specific antibodies (such as. anti-EpCAM), to facilitate specific binding.

Microliter samples containing tdEV, e.g. whole blood (unprocessed or e.g. diluted or concentrated) may be introduced in a simple microfluidic channel with dimensions similar to the electrode array. Sample size may be in the microliter or nanoliter range, or may even be tens or hundreds of picoliter. When a tdEV approaches the functionalized (nano)electrode surface, it may interact with antibodies and gets immobilized, which blocks mass transfer of the redox mediator to the electrode, resulting in an abrupt drop in the measured current (also indicated with the term "OFF signal"). A longer OFF signal indicates better specificity of the analyte entity to the probe. If the dissociation rate constant, k_{off} (*K*_{*D*×}/*kₒₙ*), is smaller, the tdEV tends to stay longer on the electrode, resulting in longer current blockage. Thus, the OFF signal time duration may give valuable information about the interaction strength of the analyte with the functionalized electrode surface. The amplitude of the current drop can also give information regarding the particle size. Since an interaction between e.g. non tdEV and the recognition element is not specific, non-tdEV may be dissociated faster on average, resulting in a shorter OFF signal period compared to the longer period of tdEVs. After breakup of antibody-antigen complex (in the order of seconds), a new sample can be injected. By introducing the sample at ~1 µl per step (volume that may contain a few tens of EVs), the total volume may be screened within ~15 minutes.

In embodiments, the invention may provide a sensor 100 for the label-free electrochemical detection of particles 10 such as tdEVs with high sensitivity and specificity, down to the single tdEV level. For specific detection, the electrode 110 may be functionalized with antibodies. One of the main challenges at such low concentrations, is the diffusion time of the particles, especially of biomarkers or biological particles, to the electrodes 110, which time for instance is relatively large for EVs. Therefore, in the invention advantage may be taken of the electrophoretic force. Owing to e.g. an oxidation reaction at the electrode, the particles 10 may be attracted onto the electrode 110, making the transport several orders faster. Other biomolecules may also be attracted to the electrode 110. Therefore, in embodiments, an antifouling layer 113 may be configured at the electrode 110 to assist in highly selective detection.

The terms "upstream" and "downstream" relate to an arrangement of items or features relative to the propagation of an element such as a particle or a fluid in a channel or light in a beam of light (during operation), wherein relative to a first position within the channel or beam, a second position in the channel or beam closer to an inlet (for the element or fluid) of the channel or respectively closer to a light generating means is "upstream", and a third position within the channel further away from the inlet or respectively further away from the light generating means "downstream".

The term "plurality" refers to two or more. Furthermore, the terms "a plurality of" and "a number of" may be used interchangeably. The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%. Moreover, the terms "about" and "approximately" may also relate to 90% or higher, such as 95% or higher, especially 99% or higher, even more especially 99.5% or higher, including 100%. For numerical values it is to be understood that the terms "substantially", "essentially", "about", and "approximately" may also relate to the range of 90% - 110%, such as 95%-105%, especially 99%-101% of the values(s) it refers to.

The term "comprise" includes also embodiments wherein the term "comprises" means "consists of". The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The term "further embodiment" and similar terms may refer to an embodiment comprising the features of the previously discussed embodiment, but may also refer to an alternative embodiment.

The devices, apparatus, or systems may herein amongst others be described during operation. As will be clear to the person skilled in the art, the invention is not limited to methods of operation, or devices, apparatus, or systems in operation.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", "include", "including", "contain", "containing" and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or a system claim, enumerating several means, several of these means may be embodied by one and the same item of hardware. The mere fact that certain measures are recited in mutually different dependent claims does not indicate that a combination of these measures cannot be used to advantage.

The disclosure also provides a control system that may control the device, apparatus, or system, or that may execute the herein described method or process. Yet further, the disclosure also provides a computer program product, when running on a computer which is functionally coupled to or comprised by the device, apparatus, or system, controls one or more controllable elements of such device, apparatus, or system.

The disclosure further applies to a device, apparatus, or system comprising one or more of the characterizing features described in the description and/or shown in the attached drawings. The disclsoure further pertains to a method or process comprising one or more of the characterizing features described in the description and/or shown in the attached drawings. Moreover, if a method or an embodiment of the method is described being executed in a device, apparatus, or system, it will be understood that the device, apparatus, or system is suitable for or configured for (executing) the method or the embodiment of the method respectively.

The various aspects discussed in this patent can be combined in order to provide additional advantages. Further, the person skilled in the art will understand that embodiments can be combined, and that also more than two embodiments can be combined.

## Claims

1. A sensor (100) for sensing a predetermined particle (10) in a fluid (11), wherein the sensor (100) comprises (i) an electrode (110) and (ii) a recognition element (112) functionally coupled to the electrode (110); wherein the electrode (110) comprises an electrode face (111) configured accessible to the fluid (11), to the predetermined particle (10) in the fluid (11), and to a redox mediator (12) in the fluid (11); wherein the recognition element (112) is configured for at least temporarily selectively binding with the predetermined particle (10), and configured to limit access of the redox mediator (12) to the electrode face (111) during the binding of the predetermined particle (10) with the recognition element (112), wherein:
- the predetermined particle (10) comprises a biological particle selected from the group comprising an extracellular vesicle (EV), a tumor-derived extracellular vesicle (tdEV), a virus, a DNA-containing particle, a particle modified with DNA, an RNA-containing particle, a particle modified with RNA, a platelet, an allergen, a bacterium, a peptide, a polypeptide, a protein, a lipoprotein, a hormone, a biopolymer, and an enzyme;
- the recognition element (112) comprises a biological recognition element (112) for the predetermined particle (10);
- the recognition element (112) is configured at the electrode face (111); and
- a characteristic dimension (d) of the electrode face (111) is selected from the range of 30-1500 nm and a size of the electrode face (111) is configured for detecting the predetermined particle (10) on a single particle level, wherein the characteristic dimension (d) of the electrode face (111) is selected to match the predetermined particle (10), and wherein the characteristic dimension (d) is selected from the group consisting of a length, a width, and an equivalent circular diameter; and
wherein the sensor comprises an array (200) of electrodes (110), wherein each electrode (110) is functionally coupled to a respective recognition element (111), wherein the array comprises at least two electrodes (110), wherein each of the electrodes (110) is configured for individually sensing a predetermined particle (10).

2. The sensor (100) according to claim 1, wherein any one of the recognition elements (112) is selected from the group comprising an antibody, a single-domain antibody, a nanobody, a knottin, a protein, an enzyme, a polypeptide, a peptide, an aptamer and a nucleic acid.

3. The sensor (100) according to any one of the preceding claims, wherein the sensor (100) comprises an electrically insulating base (120) enclosing at least part of the electrodes (110), wherein the electrically insulating base (120) comprises an insulating base face (126), configured parallel to or protruding from the electrode faces (111).

4. The sensor (100) according to claim 3, wherein the electrode faces (111) are configured recessed in the base (120).

5. The sensor (100) according to any one of the preceding claims, wherein the electrodes (110) comprise a coating (113) configured at the electrode face (111), wherein the coating (113) is configured not to block an electron transfer between the electrode face (111) and the redox mediator (12), wherein the coating (113) is configured for reducing or preventing fouling of the electrode face (111), and wherein the coating (113) further comprises the recognition element (112).

6. The sensor (100) according to any one of the preceding claims, wherein optionally at least one of the electrodes (110) of the array (200) has an electrode characteristic (119) being different from the electrode characteristic (119) of the other electrodes (110) of the array (200), wherein the electrode characteristic (119) is selected from the group consisting of a dimension (d) of the electrode face (111), the coating (113), the recognition element (112), and an electrically conductive material of the electrode (110).

7. A device (1000) for analyzing a fluid (11), wherein the device (1000) comprises an analyzing space (350) comprising the sensor (100) according to any one of the preceding claims, wherein the electrode faces (111) are configured in fluid contact with the analyzing space (350).

8. The device (1000) according to claim 7, comprising a channel (300) with a channel wall (310), wherein the channel (300) defines the analyzing space (350), and wherein the channel wall (310) comprises the electrodes (110), wherein the channel (300) is a flow through channel.

9. A system (2000) for analyzing a fluid (11), comprising the device (1000) according to any one of claims 7-8, the system (2000) further comprising a further electrode (17), wherein the further electrode (17) is configured to functionally connect to the fluid (11) in the analyzing space (350) during operation of the system (2000), the system (2000) further comprising a control system (1500), wherein the control system (1500) is configured to execute a measuring routine, wherein the measuring routine comprises:
measuring during an analyzing period an electric current through the electrodes (110) caused by a potential difference between the further electrode (17) and the respective electrode face (111),
wherein the system (2000) further comprises an electric current measuring device (16) configured to measure the electric current through the electrodes (110).

10. The system (2000) according to claim 10, wherein the system (2000) further comprises an electric power supply (15) configured for providing the potential difference between the further electrode (17) and the electrode faces (111).

11. The system (2000) according to any one of the claims 9-10, wherein the device (1000) comprises the channel (300) according to claim 8, wherein the system (2000) further comprises a fluid transport device (400) functionally connected to the channel (300), wherein the fluid transport device (400) is configured to (i) provide the fluid (11) to the analyzing space (350) and to (ii) remove the fluid (11) from the analyzing space (350) after maintaining the fluid (11) in the analyzing space (350) during the analyzing period.

12. The system (2000) according to claim 11, wherein the system (2000) is configured for providing a series of volumes (V) of the fluid (11) to the channel (300), wherein the volumes (V) of the fluid (11) are separated from each other by a separation fluid (19), wherein the system (2000) is further configured to successively execute the measuring routine for each volume (V) of the fluid (11), wherein successively (i) each volume (V) of the series of volumes (V) of the fluid (11) is provided to the analyzing space (350) and (ii) removed from the analyzing space (350) after maintaining each volume (V) in the analyzing space (350) during the analyzing period.

13. A method for analyzing a fluid (11), the method comprising:
- providing the system (2000) according to any of the claims 9-12, wherein the system (2000) comprises the electric power supply (15) according to claim 10, wherein the electrode faces (111) are functionally connected to the further electrode (17), and
during a measuring stage:
- (i) providing the fluid (11) comprising a redox mediator (12) to the analyzing space (350),
- (ii) executing a measuring routine during an analyzing period, wherein the fluid is maintained in the analyzing space during the analyzing period, and
- (iii) removing the fluid from the analyzing space again;
wherein the measuring routine comprises: providing a potential difference between the further electrode (17) and the electrode faces (111) and measuring an electric current through the respective electrode (110), as a function of time; and
wherein analyzing the fluid (11) comprises determining a presence of a predetermined particle (10) in the fluid (11), wherein the presence of the predetermined particle (10) is determined based on a minimal duration of a determined change in the measured electric current as a function of time.

14. The method according to claim 13, wherein analyzing the fluid (11) further comprises determining a concentration of the predetermined particle (10) in the fluid (11), wherein the concentration of the predetermined particle (10) is determined based on a number of determined changes over at least the minimal duration in the measured current as a function of time, relative to the analyzing period, wherein the fluid (11) comprises a fluid (11) selected from the group consisting of blood, urine, saliva, sweat, seminal fluid, cerebrospinal fluid, ascites, lymph, milk, gastric acid, lacrimal fluid, and bile.

15. The method according to any one of the claims 13-14, for analyzing a series of volumes (V) of fluid (11), wherein the system (2000) comprises the channel (300) with the channel wall (310), wherein the channel (300) defines the analyzing space (350), wherein
- the method comprising: providing a series of volumes (V) of fluid (11) comprising the redox mediator (12) to the channel (300), wherein the volumes (V) of the fluid (11) are separated from each other by a separation fluid (19), and
- wherein the measuring stage comprising: flowing the series of volumes (V) of fluid (11) comprising the redox mediator (12) through the analyzing space (350), thereby sequentially,
- (i) providing one of the volumes (V) of the series of volumes (V) to the analyzing space (350),
- (ii) executing the measuring routine during the analyzing period, wherein the respective volume (V) of fluid (11) is maintained in the analyzing space during the analyzing period, and
- (iii) removing the respective volume (V) of fluid (11) from the analyzing space (350) again, thereby providing the separation fluid (19) to the analyzing space (350);
wherein the respective volumes (V) of fluid are analyzed sequentially.

## Patentansprüche

1. Sensor (100) zum Abfühlen eines vorbestimmten Partikels (10) in einem Fluid (11), wobei der Sensor (100) (i) eine Elektrode (110) und (ii) ein Erkennungselement (112) umfasst, das funktionell an die Elektrode (110) gekoppelt ist; wobei die Elektrode (110) eine Elektrodenfläche (111) umfasst, die für das Fluid (11), das vorbestimmte Partikel (10) in dem Fluid (11) und einen Redoxmediator (12) in dem Fluid (11) zugänglich ausgestaltet ist; wobei das Erkennungselement (112) für mindestens zeitweilig selektive Bindung mit dem vorbestimmten Partikel (10) ausgestaltet ist, und ausgestaltet ist, um den Zugang des Redoxmediators (12) zu der Elektrodenfläche (111) während des Bindens des vorbestimmten Partikels (10) an das Erkennungselement (112) zu begrenzen, wobei:
- das vorbestimmte Partikel (10) ein biologisches Partikel ausgewählt aus der Gruppe umfasst, die ein extrazelluläres Vesikel (EV), ein tumorabgeleitetes extrazelluläres Vesikel (tdEV), einen Virus, ein DNA-haltiges Partikel, ein mit DNA modifiziertes Partikel, ein RNA-haltiges Partikel, ein mit RNA modifiziertes Partikel, einen Thrombozyten, ein Allergen, ein Bakterium, ein Peptid, ein Polypeptid, ein Protein, ein Lipoprotein, ein Hormon, ein Biopolymer und ein Enzym umfasst;
- das Erkennungselement (112) ein biologisches Erkennungselement (112) für das vorbestimmte Partikel (10) umfasst;
- das Erkennungselement (112) an der Elektrodenfläche (111) ausgestaltet ist; und
- eine charakteristische Dimension (d) der Elektrodenfläche (111) aus dem Bereich von 30 bis 1500 nm ausgewählt ist, und eine Größe der Elektrodenfläche (111) zum Detektieren des vorbestimmten Partikels (10) auf einem Einzelpartikelniveau ausgestaltet ist, wobei die charakteristische Dimension (d) der Elektrodenfläche (111) so ausgewählt ist, dass sie zu dem vorbestimmten Partikel (10) passt, und wobei die charakteristische Dimension (d) aus der Gruppe bestehend aus einer Länge, einer Breite und einem äquivalenten Kreisdurchmesser ausgewählt ist; und wobei der Sensor eine Gruppierung (200) von Elektroden (110) umfasst, wobei jede Elektrode (110) funktionell an ein jeweiliges Erkennungselement (111) gekoppelt ist, wobei die Gruppierung mindestens zwei Elektroden (110) umfasst, wobei jede der Elektroden (110) zum individuellen Abfühlen eines vorbestimmten Partikels (10) ausgestaltet ist.

2. Sensor (100) nach Anspruch 1, wobei jedwedes der Erkennungselemente (112) ausgewählt ist aus der Gruppe umfassend einen Antikörper, einen Einzeldomänen-Antikörper, einen Nanobody, einen Knottin, ein Protein, ein Enzym, ein Polypeptid, ein Peptid, ein Aptamer und eine Nukleinsäure.

3. Sensor (100) nach einem der vorhergehenden Ansprüche, wobei der Sensor (100) eine elektrisch isolierende Basis (120) umfasst, die mindestens einen Teil der Elektroden (110) umschließt, wobei die elektrisch isolierende Basis (120) eine isolierende Basisfläche (126) umfasst, die parallel zu den Elektrodenflächen (111) ausgestaltet ist oder aus diesen vorspringt.

4. Sensorfläche (100) nach Anspruch 3, wobei die Elektrodenflächen (111) versenkt in der Basis (120) ausgestaltet sind.

5. Sensor (100) nach einem der vorhergehenden Ansprüche, wobei die Elektroden (110) eine Beschichtung (113) umfassen, die an der Elektrodenfläche (111) ausgestaltet ist, wobei die Beschichtung (113) ausgestaltet ist, um einen Elektronentransfer zwischen der Elektrodenfläche (111) und dem Redoxmediator (12) nicht zu blockieren, wobei die Beschichtung (113) ausgestaltet ist, um Fouling (Verschmutzung) der Elektrodenfläche (111) zu reduzieren oder ihr vorzubeugen, und wobei die Beschichtung (113) des Weiteren das Erkennungselement (112) umfasst.

6. Sensor (100) nach einem der vorhergehenden Ansprüche, wobei gegebenenfalls mindestens eine der Elektroden (110) der Gruppierung (200) ein Elektrodencharakteristikum (119) hat, das sich von dem Elektrodencharakteristikum (119) der anderen Elektroden (110) der Gruppierung (200) unterscheidet, wobei das Elektrodencharakteristikum (119) ausgewählt ist aus der Gruppe bestehend aus einer Dimension (d) der Elektrodenfläche (111), der Beschichtung (113), dem Erkennungselement (112) und einem elektrisch leitfähigen Material der Elektrode (110).

7. Vorrichtung (1000) zum Analysieren eines Fluids (11), wobei die Vorrichtung (1000) einen Analyseraum (350) umfasst, der den Sensor (100) gemäß einem der vorhergehenden Ansprüche umfasst, wobei die Elektrodenflächen (111) in Fluidkontakt mit dem Analyseraum (350) ausgestaltet sind.

8. Vorrichtung (1000) nach Anspruch 7, umfassend einen Kanal (300) mit einer Kanalwand (310), wobei der Kanal (300) den Analyseraum (350) definiert, und wobei die Kanalwand (310) die Elektroden (110) umfasst, wobei der Kanal (300) ein Durchflusskanal ist.

9. System (2000) zum Analysieren eines Fluids (11), umfassend die Vorrichtung (1000) gemäß einem der Ansprüche 7 bis 8, wobei das System (2000) des Weiteren eine weitere Elektrode (17) umfasst, wobei die weitere Elektrode (17) ausgestaltet ist, um während des Betriebs des Systems (2000) funktionell mit dem Fluid (11) in dem Analyseraum (350) verbunden zu werden, wobei das System (2000) des Weiteren ein Steuersystem (1500) umfasst, wobei das Steuersystem (1500) ausgestaltet ist, um eine Messroutine auszuführen, wobei die Messroutine umfasst:
während eines Analysezeitraums Messen eines elektrischen Stroms durch die Elektroden (110) hindurch, der durch eine Potentialdifferenz zwischen der weiteren Elektrode (17) und der jeweiligen Elektrodenseite (111) bewirkt wird,
wobei das System (2000) des Weiteren eine elektrische Strommessvorrichtung (16) umfasst, die ausgestaltet ist, um den elektrischen Strom durch die Elektroden (110) hindurch zu messen.

10. System (2000) nach Anspruch 9, wobei das System (2000) des Weiteren eine elektrische Stromversorgung (15) umfasst, die ausgestaltet ist, um die Potentialdifferenz zwischen der weiteren Elektrode (17) und den Elektrodenflächen (111) bereitzustellen.

11. System (2000) nach einem der Ansprüche 9 bis 10, wobei die Vorrichtung (1000) den Kanal (300) gemäß Anspruch 8 umfasst, wobei das System (2000) des Weiteren eine Fluidtransportvorrichtung (400) umfasst, die funktionell mit dem Kanal (300) verbunden ist, wobei die Fluidtransportvorrichtung (400) ausgestaltet ist, um (i) dem Analyseraum (350) das Fluid (11) bereitzustellen, und (ii) das Fluid (11) aus dem Analyseraum (350) zu entfernen, nachdem das Fluid (11) während des Analysezeitraums in dem Analyseraum (350) gehalten wurde.

12. System (2000) nach Anspruch 11, wobei das System (2000) ausgestaltet ist, um dem Kanal (300) eine Reihe von Volumina (V) des Fluids (11) bereitzustellen, wobei die Volumina (V) des Fluids (11) durch ein Trennfluid (19) voneinander getrennt sind, wobei das System (2000) des Weiteren ausgestaltet ist, um nacheinander die Messroutine für jedes Volumen (V) des Fluids (11) auszuführen, wobei nacheinander (i) jedes Volumen (V) der Reihe von Volumina (V) des Fluids (11) dem Analyseraum (350) bereitgestellt wird und (ii) aus dem Analyseraum (350) entfernt wird, nachdem jedes Volumen (V) in dem Analyseraum (350) während des Analysezeitraums gehalten wurde.

13. Verfahren zum Analysieren eines Fluids (11), wobei das Verfahren umfasst:
- Bereitstellen des Systems (2000) nach einem der Ansprüche 9 bis 12, wobei das System (2000) die elektrische Stromversorgung (15) gemäß Anspruch 10 umfasst, wobei die Elektrodenflächen (111) funktionell mit der weiteren Elektrode (17) verbunden sind, und
während einer Messstufe:
- (i) Bereitstellen des Fluids (11), umfassend einen Redoxmediator (12), an den Analyseraum (350),
- (ii) Ausführen einer Messroutine während eines Analysezeitraums, wobei das Fluid während des Analysezeitraums in dem Analyseraum gehalten wird, und
- (iii) erneutes Entfernen des Fluids aus dem Analyseraum;
wobei die Messroutine umfasst: Bereitstellen einer Potentialdifferenz zwischen der weiteren Elektrode (17) und den Elektrodenflächen (111), und Messen eines elektrischen Stroms durch die jeweilige Elektrode (110) hindurch als Funktion der Zeit; und
wobei Analysieren des Fluids (11) Bestimmen einer Anwesenheit eines vorbestimmten Partikels (10) in dem Fluid (11) umfasst, wobei die Anwesenheit des vorbestimmten Partikels (10) basierend auf einer Mindestdauer einer bestimmten Veränderung in dem gemessenen elektrischen Strom als Funktion der Zeit bestimmt wird.

14. Verfahren nach Anspruch 13, wobei Analysieren des Fluids (11) des Weiteren Bestimmen einer Konzentration des vorbestimmten Partikels (10) in dem Fluid (11) umfasst, wobei die Konzentration des vorbestimmten Partikels (10) basierend auf einer Anzahl der bestimmten Änderungen über mindestens der Mindestdauer in dem gemessenen Strom als Funktion der Zeit relativ zu dem Analysezeitraum bestimmt wird, wobei das Fluid (11) ein Fluid (11) ausgewählt aus der Gruppe bestehend aus Blut, Urin, Speichel, Schweiß, Samenflüssigkeit, Zerebrospinalflüssigkeit, Aszites, Lymphe, Milch, Magensäure, Tränenflüssigkeit und Galle umfasst.

15. Verfahren nach einem der Ansprüche 13 bis 14 zum Analysieren einer Reihe von Volumina (V) an Fluid (11), wobei das System (2000) den Kanal (300) mit einer Kanalwand (310) umfasst, wobei der Kanal (300) den Analyseraum (350) definiert, wobei
- das Verfahren umfasst: Bereitstellen einer Reihe von Volumina (V) an Fluid (11), umfassend den Redoxmediator (12), an den Kanal (300), wobei die Volumina (V) des Fluids (11) durch ein Trennfluid (19) voneinander getrennt sind, und
- wobei die Messstufe umfasst: Fließenlassen der Reihe an Volumina (V) des Fluids, umfassend den Redoxmediator (12), durch den Analyseraum (350), dadurch aufeinander folgend:
- (i) Bereitstellen von einem der Volumina (V) der Reihe an Volumina (V) an den Analyseraum (350),
- (ii) Ausführen der Messroutine während des Analysezeitraums, wobei das jeweilige Volumen (V) des Fluids (11) während des Analysezeitraums in dem Analyseraum gehalten wird, und
- (iii) erneutes Entfernen des jeweiligen Volumens (V) des Fluids (11) aus dem Analyseraum (350), wodurch dem Analyseraum (350) das Trennfluid (19) bereitgestellt wird;
wobei die jeweiligen Volumina (V) an Fluid aufeinander folgend analysiert werden.

## Revendications

1. Capteur (100) permettant de détecter une particule prédéterminée (10) dans un fluide (11), le capteur (100) comprenant (i) une électrode (110) et (ii) un élément de reconnaissance (112) fonctionnellement couplé à l'électrode (110) ; dans lequel l'électrode (110) comprend une face d'électrode (111) configurée pour être accessible par le fluide (11), par la particule prédéterminée (10) dans le fluide (11) et par un médiateur redox (12) dans le fluide (11) ; dans lequel l'élément de reconnaissance (112) est configuré pour se lier sélectivement, au moins temporairement, à la particule prédéterminée (10), et configuré pour limiter l'accès du médiateur redox (12) à la face d'électrode (111) pendant la liaison de la particule prédéterminée (10) avec l'élément de reconnaissance (112), dans lequel :
- la particule prédéterminée (10) comprend une particule biologique choisie dans le groupe comprenant une vésicule extracellulaire (VE), une vésicule extracellulaire dérivée d'une tumeur (tdEV), un virus, une particule contenant de l'ADN, une particule modifiée avec de l'ADN, une particule contenant de l'ARN, une particule modifiée avec de l'ARN, une plaquette, un allergène, une bactérie, un peptide, un polypeptide, une protéine, une lipoprotéine, une hormone, un biopolymère et une enzyme ;
- l'élément de reconnaissance (112) comprend un élément de reconnaissance biologique (112) pour la particule prédéterminée (10) ;
- l'élément de reconnaissance (112) est configuré au niveau de la face d'électrode (111) ; et
- une dimension caractéristique (d) de la face d'électrode (111) est choisie dans la plage de 30 à 1 500 nm et une taille de la face d'électrode (111) est configurée pour détecter la particule prédéterminée (10) sur un niveau de particule unique, dans lequel la dimension caractéristique (d) de la face d'électrode (111) est choisie pour correspondre à la particule prédéterminée (10), et dans lequel la dimension caractéristique (d) est choisie dans le groupe constitué par une longueur, une largeur et un diamètre circulaire équivalent ; et
dans lequel le capteur comprend un réseau (200) d'électrodes (110), dans lequel chaque électrode (110) est couplée fonctionnellement à un élément de reconnaissance (111) respectif, dans lequel le réseau comprend au moins deux électrodes (110), dans lequel chacune des électrodes (110) est configurée pour détecter individuellement une particule prédéterminée (10).

2. Capteur (100) selon la revendication 1, dans lequel un quelconque des éléments de reconnaissance (112) est choisi dans le groupe comprenant un anticorps, un anticorps à domaine unique, un nanocorps, un nœud de cystine inhibiteur, une protéine, une enzyme, un polypeptide, un peptide, un aptamère et un acide nucléique.

3. Capteur (100) selon l'une quelconque des revendications précédentes, le capteur (100) comprenant une base électro-isolante (120) entourant au moins une partie des électrodes (110), dans lequel la base électro-isolante (120) comprend une face de base isolante (126), configurée parallèlement aux faces d'électrode (111) ou dépassant de celles-ci.

4. Capteur (100) selon la revendication 3, dans lequel les faces d'électrode (111) sont configurées en creux dans la base (120).

5. Capteur (100) selon l'une quelconque des revendications précédentes, dans lequel les électrodes (110) comprennent un revêtement (113) configuré au niveau de la face d'électrode (111), dans lequel le revêtement (113) est configuré pour ne pas bloquer un transfert d'électrons entre la face d'électrode (111) et le médiateur redox (12), dans lequel le revêtement (113) est configuré pour réduire ou empêcher l'encrassement de la face d'électrode (111), et dans lequel le revêtement (113) comprend en outre l'élément de reconnaissance (112).

6. Capteur (100) selon l'une quelconque des revendications précédentes, dans lequel, éventuellement, au moins une des électrodes (110) du réseau (200) présente une caractéristique d'électrode (119) différente de la caractéristique d'électrode (119) des autres électrodes (110) du réseau (200), dans lequel la caractéristique d'électrode (119) est choisie dans le groupe constitué par une dimension (d) de la face d'électrode (111), le revêtement (113), l'élément de reconnaissance (112) et un matériau électroconducteur de l'électrode (110).

7. Dispositif (1000) permettant d'analyser un fluide (11), le dispositif (1000) comprenant un espace d'analyse (350) comprenant le capteur (100) selon l'une quelconque des revendications précédentes, dans lequel les faces d'électrode (111) sont configurées en contact fluidique avec l'espace d'analyse (350).

8. Dispositif (1000) selon la revendication 7, comprenant un canal (300) doté d'une paroi de canal (310), dans lequel le canal (300) définit l'espace d'analyse (350), et dans lequel la paroi de canal (310) comprend les électrodes (110), dans lequel le canal (300) est un canal d'écoulement traversant.

9. Système (2000) permettant d'analyser un fluide (11), comprenant le dispositif (1000) selon l'une quelconque des revendications 7 et 8, le système (2000) comprenant en outre une électrode supplémentaire (17), dans lequel l'électrode supplémentaire (17) est configurée pour se raccorder fonctionnellement au fluide (11) dans l'espace d'analyse (350) pendant le fonctionnement du système (2000), le système (2000) comprenant en outre un système de commande (1500), dans lequel le système de commande (1500) est configuré pour exécuter une routine de mesure, dans lequel la routine de mesure comprend :
la mesure, pendant une période d'analyse, d'un courant électrique à travers les électrodes (110) causé par une différence de potentiel entre l'électrode supplémentaire (17) et la face d'électrode respective (111),
dans lequel le système (2000) comprend en outre un dispositif de mesure de courant électrique (16) configuré pour mesurer le courant électrique à travers les électrodes (110).

10. Système (2000) selon la revendication 9, le système (2000) comprenant en outre une alimentation électrique (15) configurée pour fournir la différence de potentiel entre l'électrode supplémentaire (17) et les faces d'électrode (111).

11. Système (2000) selon l'une quelconque des revendications 9 et 10, dans lequel le dispositif (1000) comprend le canal (300) selon la revendication 8, le système (2000) comprenant en outre un dispositif de transport de fluide (400) fonctionnellement raccordé au canal (300), dans lequel le dispositif de transport de fluide (400) est configuré pour (i) fournir le fluide (11) à l'espace d'analyse (350) et pour (ii) retirer le fluide (11) de l'espace d'analyse (350) après avoir maintenu le fluide (11) dans l'espace d'analyse (350) pendant la période d'analyse.

12. Système (2000) selon la revendication 11, le système (2000) étant configuré pour fournir une série de volumes (V) du fluide (11) au canal (300), dans lequel les volumes (V) du fluide (11) sont séparés les uns des autres par un fluide de séparation (19), le système (2000) étant en outre configuré pour exécuter successivement la routine de mesure pour chaque volume (V) du fluide (11), dans lequel successivement (i) chaque volume (V) de la série de volumes (V) du fluide (11) est fourni à l'espace d'analyse (350) et (ii) retiré de l'espace d'analyse (350) après avoir maintenu chaque volume (V) dans l'espace d'analyse (350) pendant la période d'analyse.

13. Procédé permettant d'analyser un fluide (11), le procédé comprenant :
- la fourniture du système (2000) selon l'une quelconque des revendications 9 à 12, dans lequel le système (2000) comprend l'alimentation électrique (15) selon la revendication 10, dans lequel les faces d'électrode (111) sont connectées fonctionnellement à l'électrode supplémentaire (17), et
lors d'une étape de mesure :
- (i) la fourniture du fluide (11) comprenant un médiateur redox (12) à l'espace d'analyse (350),
- (ii) l'exécution d'une routine de mesure pendant une période d'analyse, dans lequel le fluide est maintenu dans l'espace d'analyse pendant la période d'analyse, et
- (iii) le retrait, une fois encore, du fluide de l'espace d'analyse ;
dans lequel la routine de mesure comprend : la fourniture d'une différence de potentiel entre l'électrode supplémentaire (17) et les faces d'électrode (111) et la mesure d'un courant électrique à travers l'électrode respective (110), en fonction du temps ; et
dans lequel l'analyse du fluide (11) comprend la détermination de la présence d'une particule prédéterminée (10) dans le fluide (11), dans lequel la présence de la particule prédéterminée (10) est déterminée sur la base d'une durée minimale d'une variation déterminée dans le courant électrique mesuré en fonction du temps.

14. Procédé selon la revendication 13, dans lequel l'analyse du fluide (11) comprend en outre la détermination d'une concentration de la particule prédéterminée (10) dans le fluide (11), dans lequel la concentration de la particule prédéterminée (10) est déterminée sur la base d'un certain nombre de changements déterminés sur au moins la durée minimale dans le courant mesuré en fonction du temps, par rapport à la période d'analyse, dans lequel le fluide (11) comprend un fluide (11) choisi dans le groupe constitué par du sang, de l'urine, de la salive, de la sueur, du liquide séminal, du liquide céphalorachidien, une ascite, de la lymphe, du lait, de l'acide gastrique, du liquide lacrymal et de la bile.

15. Procédé selon l'une quelconque des revendications 13 et 14, permettant d'analyser une série de volumes (V) de fluide (11), dans lequel le système (2000) comprend le canal (300) doté de la paroi de canal (310), dans lequel le canal (300) définit l'espace d'analyse (350), dans lequel
- le procédé comprenant : la fourniture d'une série de volumes (V) de fluide (11) comprenant le médiateur redox (12) au canal (300), dans lequel les volumes (V) du fluide (11) sont séparés les uns des autres par un fluide de séparation (19), et
- dans lequel l'étape de mesure comprenant : l'écoulement de la série de volumes (V) de fluide (11) comprenant le médiateur redox (12) à travers l'espace d'analyse (350), de ce fait, de manière séquentielle,
- (i) la fourniture de l'un des volumes (V) de la série de volumes (V) à l'espace d'analyse (350),
- (ii) l'exécution de la routine de mesure pendant la période d'analyse, dans lequel le volume respectif (V) de fluide (11) est maintenu dans l'espace d'analyse pendant la période d'analyse, et
- (iii) le retrait, une fois encore, du volume respectif (V) de fluide (11) de l'espace d'analyse (350), ce qui fournit le fluide de séparation (19) à l'espace d'analyse (350) ;
dans lequel les volumes respectifs (V) de fluide sont analysés séquentiellement.
